# EUROPEAN PATENT APPLICATION

(11) **EP 2 682 384 A1**
(43) Date of publication of application: **08.01.2014**
(21) Application number: 12747598.6
(22) Date of filing: 19.01.2012
(51) Int. Cl.: C07C 251/24, A61K 31/135, A61K 31/28, A61K 31/295, A61K 31/315, A61P 23/02, A61P 35/00, C07F 11/00, C07F 13/00, C07F 15/02, C07F 15/06

(54) **AUTO-MAGNETIC METAL SALEN COMPLEX COMPOUND**

(30) Priority: 15.02.2011 JP 2011030056
(71) Applicant: IHI Corporation, Tokyo 135-8710 (JP); Ishikawa, Yoshihiro, Tokyo 160-0022 (JP)
(72) Inventor: ISHIKAWA, Yoshihiro, Tokyo 160-0022 (JP); EGUCHI, Haruki, Tokyo 135-8710 (JP)
(74) Representative: MERH-IP Matias Erny Reichl Hoffmann
(86) International application number: PCT/JP2012/051079
(87) International publication number: WO 2012/111380

(57) **Abstract**

The molecular structures of metal-salen complexes which exerts pharmacological effects are clarified and the metal-salen complexes having such molecular structures and their derivatives are provided. A metal-salen complex compound is characterized in that a metal atom part in each of multiple molecules of the metal-salen complex or its derivative is multimerized via water.

## Description

### [Technical Field]

The present invention relates to a self-magnetic metal-salen complex compound.

### [Background Art]

Generally, when a drug is administered to a living body, it reaches an affected site and exerts its pharmacological effects at that affected site, thereby exerting its therapeutic effects. On the other hand, even if the drug reaches tissue other than the affected site (that is, normal tissue), it will not be therapeutic.

Therefore, how to guide the drug to the affected site is important. A technique to guide the drug to the affected site is called drug delivery, which has been actively studied and developed recently.

This drug delivery has at least two advantages. One advantage is that a sufficiently high drug concentration can be obtained at the affected site tissue. Pharmacological effects will not be seen unless the drug concentration at the affected site is a constant value or more. The therapeutic effects cannot be expected if the concentration is low. The second advantage is that the drug is guided to only the affected site tissue and, therefore, adverse reactions to the normal tissue can be inhibited.

Such drug delivery is most effective for a cancer treatment by antitumor agents. Most antitumor agents inhibit the cell growth of cancer cells which divide actively, so that the antitumor agents will also inhibit the cell growth of even the normal tissue in which cells divide actively, such as bone marrow, hair roots, or alimentary canal mucosa.

Therefore, cancer patients to whom the antitumor agents are administered suffer adverse reactions such as anemia, hair loss, and vomiting. Since such adverse reactions impose heavy burdens on the patients, the dosage needs to be limited, thereby causing a problem of incapability to sufficiently obtain the pharmacological effects of the antitumor agents.

Alkyl antineoplastic drugs among such antineoplastic drugs are a generic term for antitumor agents having the ability to combine an alkyl group (-CH2-CH2-) with, for example, a nuclei acid protein. It alkylates DNA and inhibits DNA replication, causing cell death. This action works regardless of cell cycles, also works on cells of the Go period, has a strong effect on cells which grow actively, and tends to damage, for example, bone marrow, alimentary canal mucosa, germ cells, or hair roots.

Moreover, antimetabolite antineoplastic drugs are compounds having structures similar to those of nucleic acids or metabolites in a protein synthesis process, impairs cells by, for example, inhibiting synthesis of the nucleic acids, and specifically acts on cells of a mitotic period.

Furthermore, antitumor antibiotics are chemical substances produced by microorganisms, have actions such as DNA synthesis inhibition and DNA strand breaking, and exhibit antitumor activity.

Also, microtubule inhibitors have antitumor effects by directly acting on microtubules that serve important roles to maintain normal functions of cells, for example, by forming spindles during cell division, locating cell organelles, and transporting substances. The microtubule inhibitors act on cells, which divide actively, and nerve cells.

Moreover, platinum preparations inhibit DNA synthesis by forming DNA strands, interchain bonds, or DNA protein bonds. Cisplatin is a representative drug, but it causes severe nephropathia and requires a large amount of fluid replacement.

Furthermore, parahormone antineoplastic drugs are effective against hormone-dependent tumors. Female hormones or anti-androgen drugs are administered to an androgen-dependent prostatic cancer.

Also, molecular targeted drugs are used for a treatment targeted at molecules that correspond to molecular biological characters specific to respective malignant tumors.

Moreover, topoisomerase inhibitors are enzymes for temporarily generating breaks in DNA and changing the number of tangles of DNA strands. A topoisomerase inhibitor I is an enzyme that generates breaks in one strand of a circular DNA, lets the other strand pass, and then closes the breaks; and a topoisomerase inhibitor II temporarily breaks both the two strands of the circular DNA, lets other two DNA strands pass between the former two strands, and reconnects the broken strands.

Furthermore, nonspecific immunopotentiators inhibit an increase of cancer cells by activating the immune system.

Topical anesthetics also have the advantage of drug delivery. The topical anesthetics are used to treat topical itches and pains of, for example, mucosa or skin caused by hemorrhoidal disease, stomatitis, gum disease, cavities, tooth extraction, or operations. Lidocaine (product name: xylocaine) is known as a representative topical anesthetic; however, lidocaine is faster-acting, but has an antiarrhythmic effect. Furthermore, if lidocaine which is an anesthetic is injected into the spinal fluid when giving spinal anesthesia, lidocaine will spread through the spinal fluid; and in a worst-case scenario, there is fear that lidocaine might reach a cervical part of the spinal cord and thereby cause a respiratory function to stop and bring about critical adverse effects.

An example of a specific method for the drug delivery is the use of a carrier. This is to load the carrier, which tends to concentrate on the affected site, with the drug and have the carrier carry the drug to the affected site.

A promising candidate of the carrier is a magnetic substance and there is a suggested method of attaching the carrier, which is the magnetic substance, to the drug and allowing the carrier to be accumulated at the affected site by a magnetic field (see, for example, Patent Literature 1).

However, when using the magnetic substance carrier as the carrier, it has been found that it is difficult to aurally administer the magnetic substance carrier, molecules of the carrier are generally giant, and there are technical problems about binding strength and affinity between the carrier and the drug molecules; and it is originally difficult to achieve the practical use of the magnetic substance carrier.

Therefore, the inventors of the present invention suggested a topical anesthetic in which side chains for giving positive or negative spin charge density are bonded to a basic skeleton of an organic compound, and which has suitability as a whole insofar as the topical anesthetic is guided, by means of magnetic sharing by an external magnetic field; and if the topical anesthetic is applied to a human body or an animal, it is retained in an area where a magnetic field is applied topically by the magnetic field outside the body and the medicinal effects that the topical anesthetic originally has are exerted on the area. The above-mentioned publication describes the iron-salen complex as an example of such a drug (see Patent Literature 2).

Furthermore, literature including reviews of organic magnetic substances (see, for example, Non Patent Literature 1) and literature describing substitution of platinum contained in cisplatin with another element (see, for example, Non Patent Literature 2) are issued.

### [Citation List]

### [Patent Literature]

[Patent Literature 1] JP-A-2001-10978
[Patent Literature 2] WO2008/001851

### [Non Patent Literature]

[Non Patent Literature 1] Hiizu Iwamura, Science, Feb. 1989 issue, p.p. 76-88
[Non Patent Literature 2] Krsity Cochran et al., Structural Chemistry, 13(2002), p.p. 133-140

### [Summary of Invention]

### [Problems to be Solved by the Invention]

However, regarding the methods and compounds described in Patent Literatures 1 and 2, the molecular structures of metal-salen complexes which exert pharmacological effects are not necessarily clear. Furthermore, neither Non Patent Literature 1 nor 2 notes that drugs themselves become magnetic.

The present invention was devised in light of such circumstances and it is an object of the invention to clarify the molecular structures of metal-salen complexes, which exerts pharmacological effects, and provide metal-salen complexes, which have such molecular structures and are magnetic themselves, and their derivatives.

### [Means for Solving the Problems]

In order to achieve the above-described object, the present invention provides a metal-salen complex compound regarding which each of multiple molecules of a metal-salen complex or its derivatives is multimerized via water.

The metal-salen complex compound can contain the metal-salen complex or a dimmer of the metal-salen complex.

Moreover, regarding the metal-salen complex compound, the multimerized metal-salen complex or derivatives of such metal-salen complex can be self-magnetic.

A preferred embodiment of the present invention is a self-magnetic metal-salen complex represented by the following chemical formula (I) and its derivatives 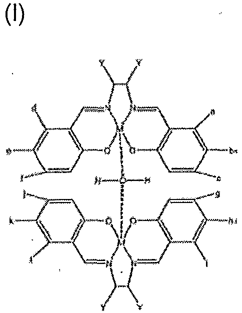 where regarding the chemical formula (I), M represents Fe, Cr, Mn, Co, Ni, Mo, Ru, Rh, Pd, W, Re, Os, Ir, Pt, Nd, Sm, Eu, or Gd, and each of a to f and Y is hydrogen or any one of the following (1) to (7):

(1) -CO₂Me;
(2) -CO(OCH₂CH₂)₂OCH₃;
(3) 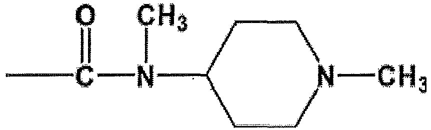
(4) 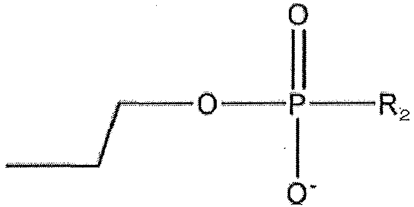 (where regarding formula (4), R₂ represents a plurality of nucleic acids which are combined together and are formed of adenine, guanine, thymine, cytosine, or uracil);
(5) -NHCOH, -NH₂, -NHR₁, or -NR₁R₂
   (were regarding (5) above, R₁ and R₂ are alkyl or alkane with the same carbon number or the carbon number from 1 to 6);
(6) -NHR₃-, -NHCOR₃, or -R₃
   (where regarding (6) above, R₃ represents a substituent bound as a result of desorption of hydrogen or a functional group such as a hydroxyl group or the like; and
(7) halogen atoms.

Regarding (6) above, charge transfer of R₃ should preferably be less than 0.5 electrons. Also, R₃ is any one of compounds represented by the following formulas (8) to (34):

(8) ibuprofen piconol, phenylpropionic acid analgesic/anti-inflammatory 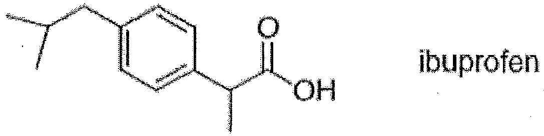
(9) mefenamic, anthranilic-acid anti-inflammatory analgesic 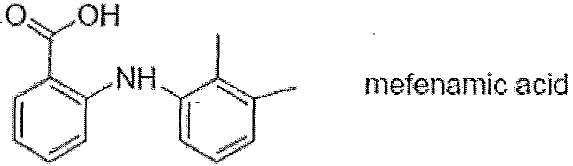
(10) drug for treating hyperlipemia 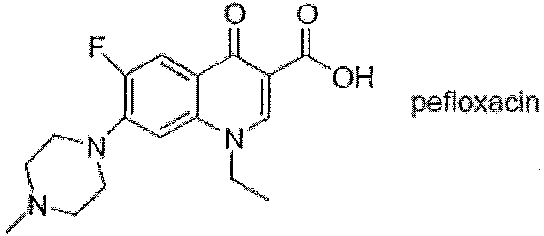
(11) antibacterial 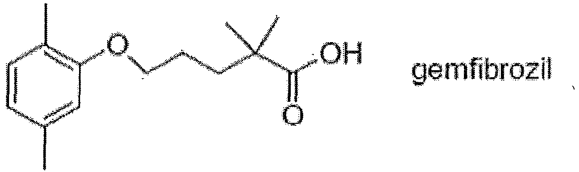
(12) fluorochrome (rhodamine) 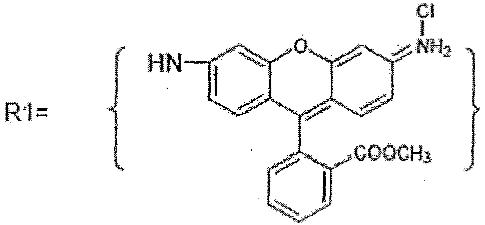
(13) hormone (estrogen) 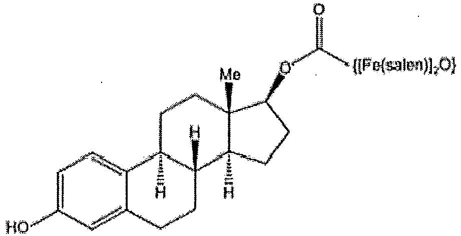
(14) hormone (estrogen) 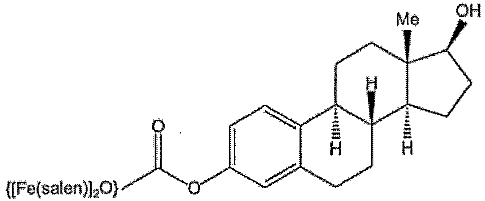
(15) Taxol (paclitaxel) 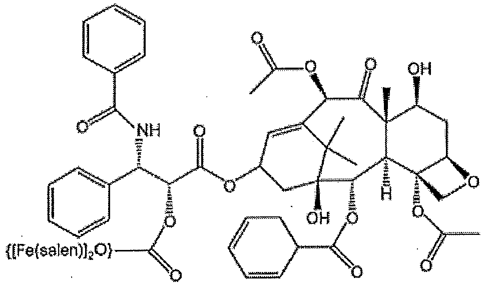
(16) amino acid (glycine) 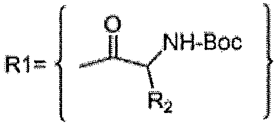
(17) amino acid (alanine) 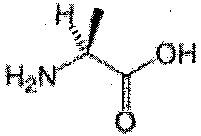
(18) amino acid (arginine) 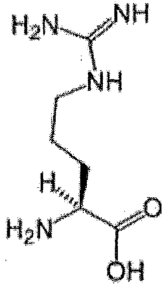
(19) amino acid (asparagine) 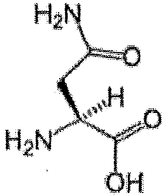
(20) amino acid (aspartic acid) 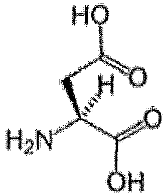
(21) amino acid (cysteine) 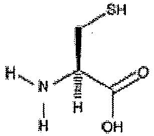
(22) amino acid (glutamic acid) 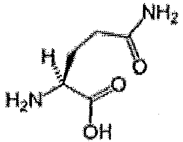
(23) amino acid (histidine) 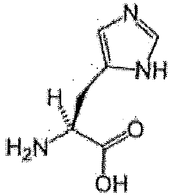
(24) amino acid (isoleucine) 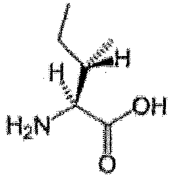
(25) amino acid (leucine) 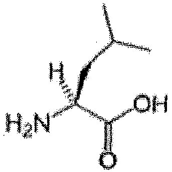
(26) amino acid (lysine) 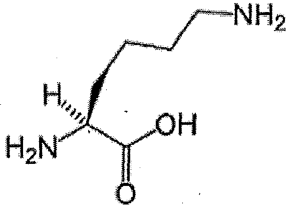
(27) amino acid (methionine) 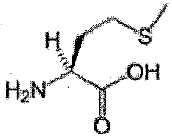
(28) amino acid (phenylalanine) 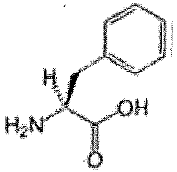
(29) amino acid (proline) 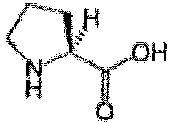
(30) amino acid (serine) 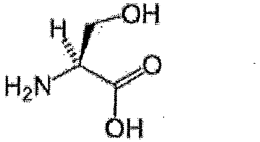
(31) amino acid (threonine) 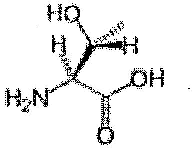
(32) amino acid (tryptophan) 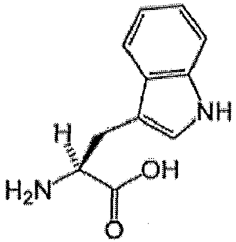
(33) amino acid (tyrosine) 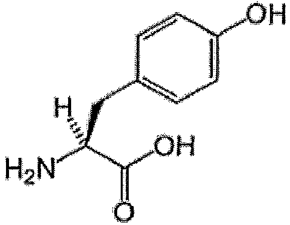
(34) amino acid (valine).

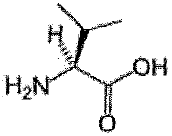

Furthermore, the present invention provides a local anesthetic having a self-magnetic metal-salen complex compound wherein R₃ is a substituent represented by any of the following formulas (35) to (45) obtained as a result of desorption of hydrogen from a compound which has a methyl group and whose charge transfer is less than 0.5 electors (e):

(35) general name: lidocaine 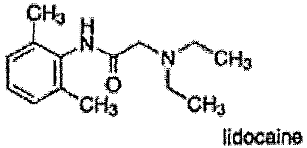
(36) general name: ethyl aminobenzoic acid 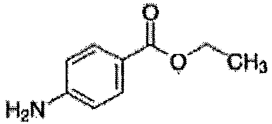
(37) general name: oxybuprocaine hydrochloride 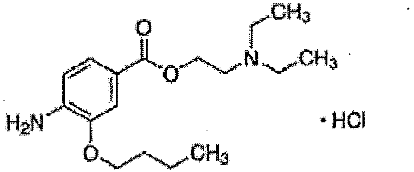
(38) general name: oxethazaine 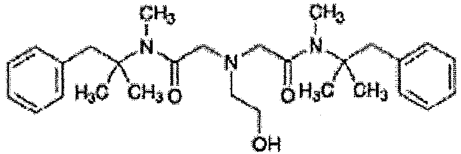
(39) general name: dibucaine 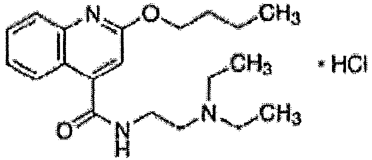
(40) general name: ethylpiperidinoacetylaminobenzoate 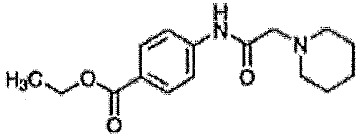
(41) general name: procaine 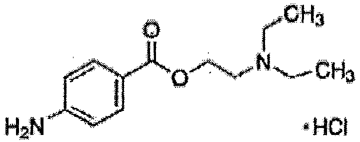
(42) general name: mepivacaine 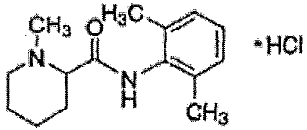
(43) general name: p-butylaminobenzoyldiethylaminoethyl hydrochloride 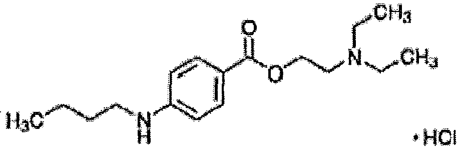
(44) general name: bupivacaine hydrochloride 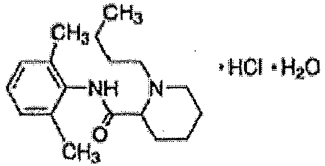
(45) general name: ropivacaine hydrochloride hydrate.

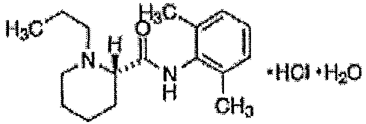

Furthermore, the present invention provides an antineoplastic drug having a self-magnetic metal-salen complex compound wherein R₃ is any one of compounds represented by the following formulas (46) to (110), which combines with a main skeleton of the compound of the above formula I via a linking group part obtained as a result of desorption of hydrogen (however, with the compound (90), a cyano group (-CN) is the linking group):

(46) general name: ifosfamide, alkyl antineoplastic drug 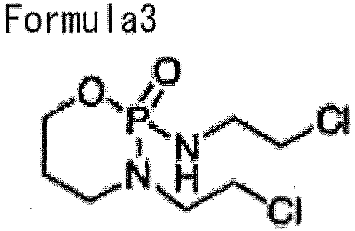
(47) general name: cyclophosphamide, alkyl antineoplastic drug 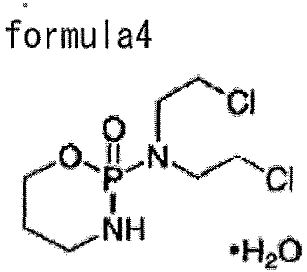
(48) general name: dacarbazine, alkyl antineoplastic drug 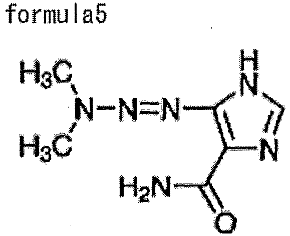
(49) general name: busulfan, alkyl antineoplastic drug 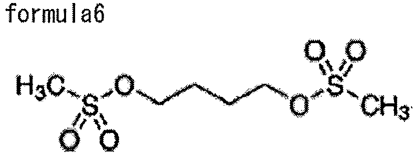
(50) general name: melphalan, alkyl antineoplastic drug 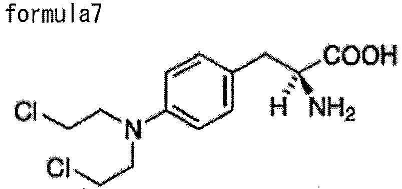
(51) general name: ranimustine, alkyl antineoplastic drug 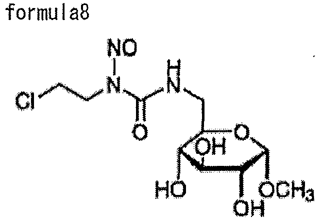
(52) general name: estramustine sodium phosphate, alkyl antineoplastic drug 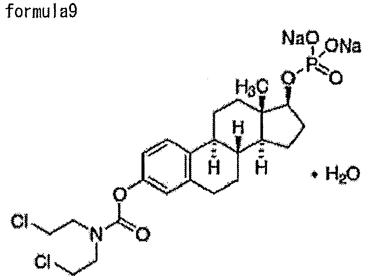
(53) general name: nimustine hydrochloride, alkyl antineoplastic drug 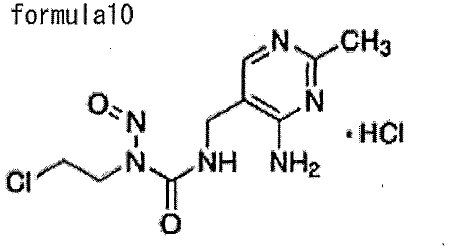
(54) general name: enocitabine, antimetabolite antineoplastic drug 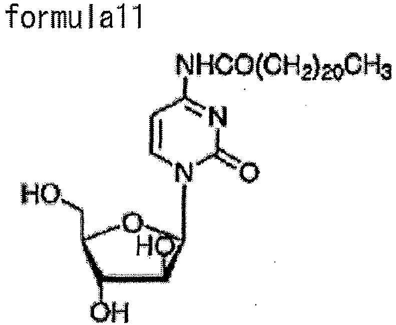
(55) general name: capecitabine, antimetabolite antineoplastic drug 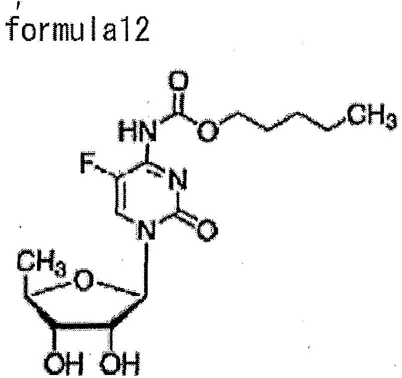
(56) general name: carmofur, antimetabolite antineoplastic drug 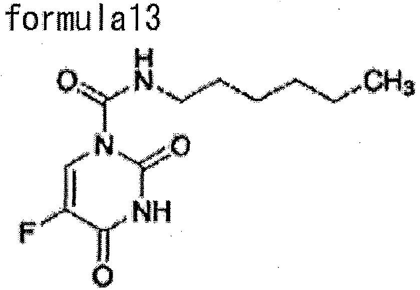
(57) general name: gimeracil, antimetabolite antineoplastic drug 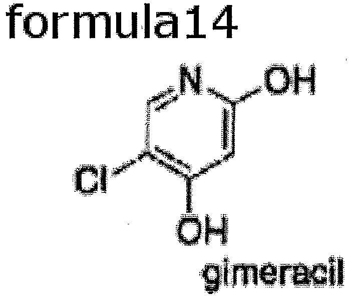
(58) general name: oteracil potassium, antimetabolite antineoplastic drug 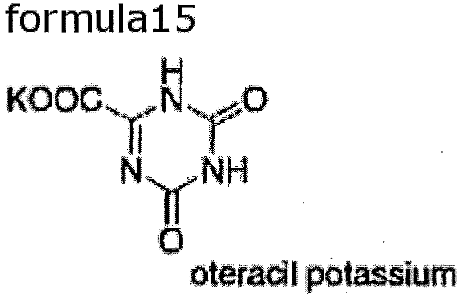
(59) general name: cytarabine, antimetabolite antineoplastic drug 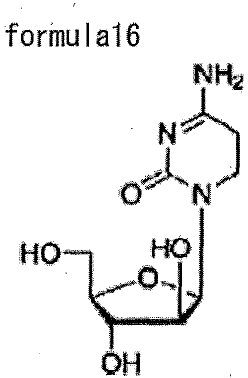
(60) general name: cytarabine ocfosfate, antimetabolite antineoplastic drug 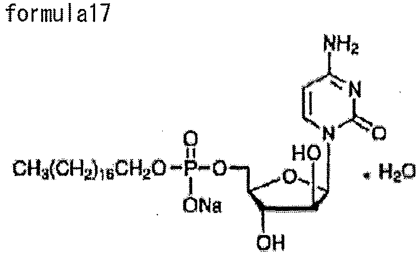
(61) general name: tegafur, antimetabolite antineoplastic drug 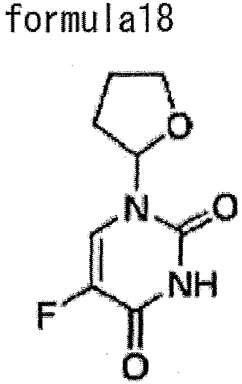
(62) general name: doxifluridine, antimetabolite antineoplastic drug 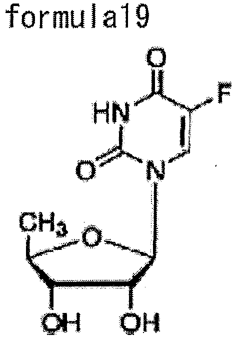
(63) general name: hydroxycarbamide, antimetabolite antineoplastic drug 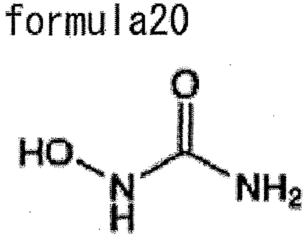
(64) general name: fluorouracil, antimetabolite antineoplastic drug 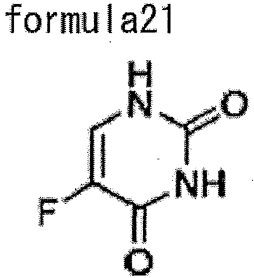
(65) general name: mercaptopurine hydrate, antimetabolite antineoplastic drug 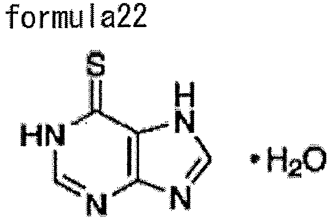
(66) general name: fludarabine phosphate, antimetabolite antineoplastic drug forumula23 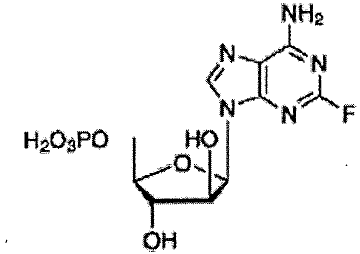
(67) general name: gemcitabine hydrochloride, antimetabolite antineoplastic drug 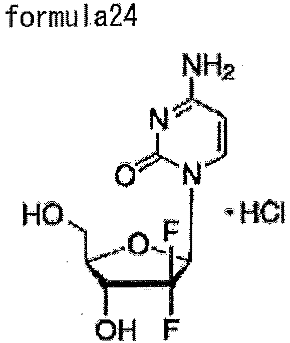
(68) general name: actinomycin-D, antitumor antibiotic 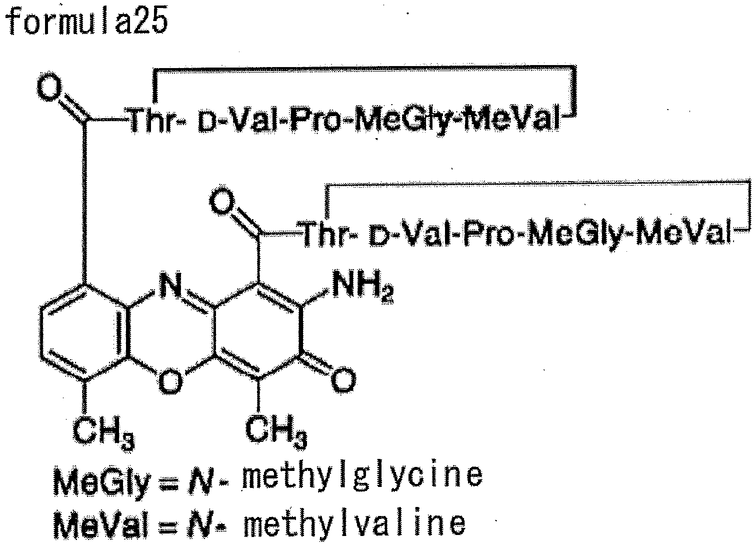
(69) general name: aclarubicin hydrochloride, antitumor antibiotic 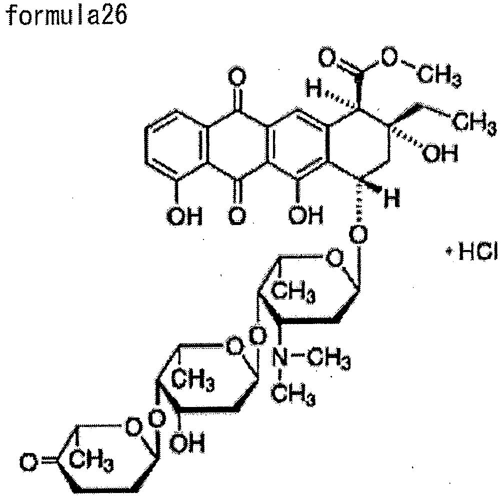
(70) general name: idarubicin hydrochloride, antitumor antibiotic 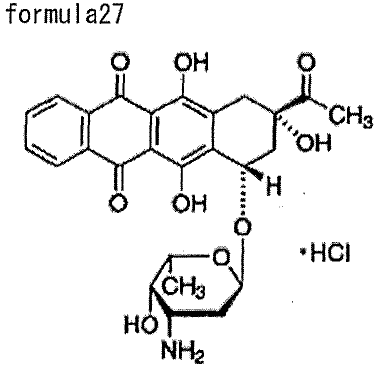
(71) general name: epirubicin hydrochloride, antitumor antibiotic 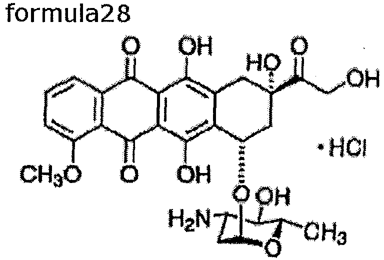
(72) general name: zinostatin stimalamer, antitumor antibiotic 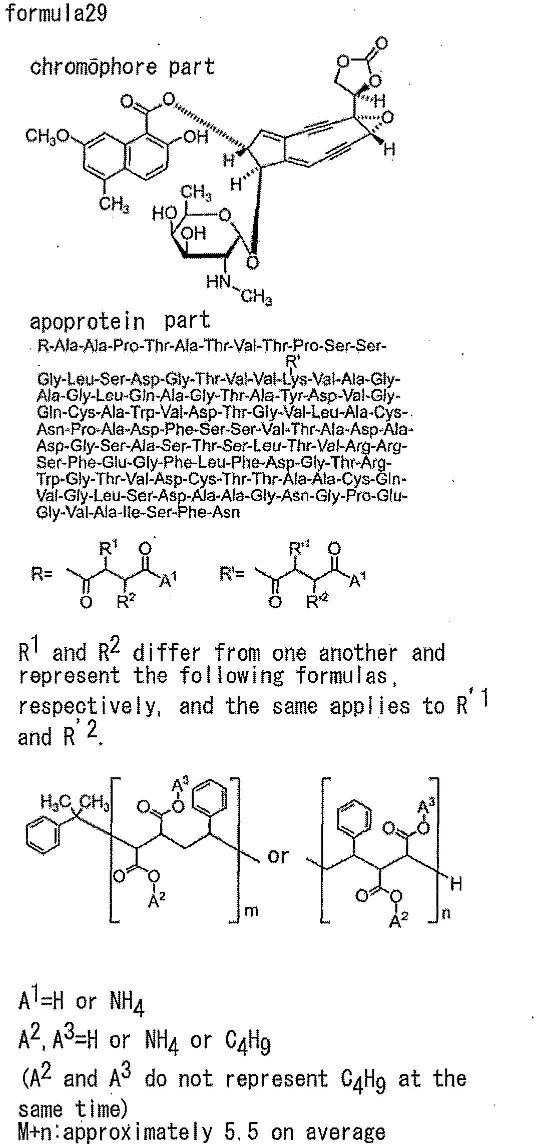
(73) general name: daunorubicin hydrochloride, antitumor antibiotic 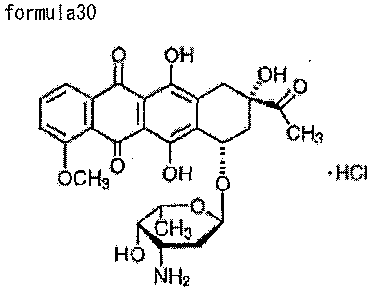
(74) general name: doxorubicin hydrochloride, antitumor antibiotic 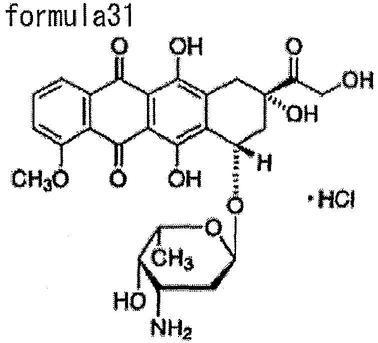
(75) general name: bleomycin hydrochloride, antitumor antibiotic 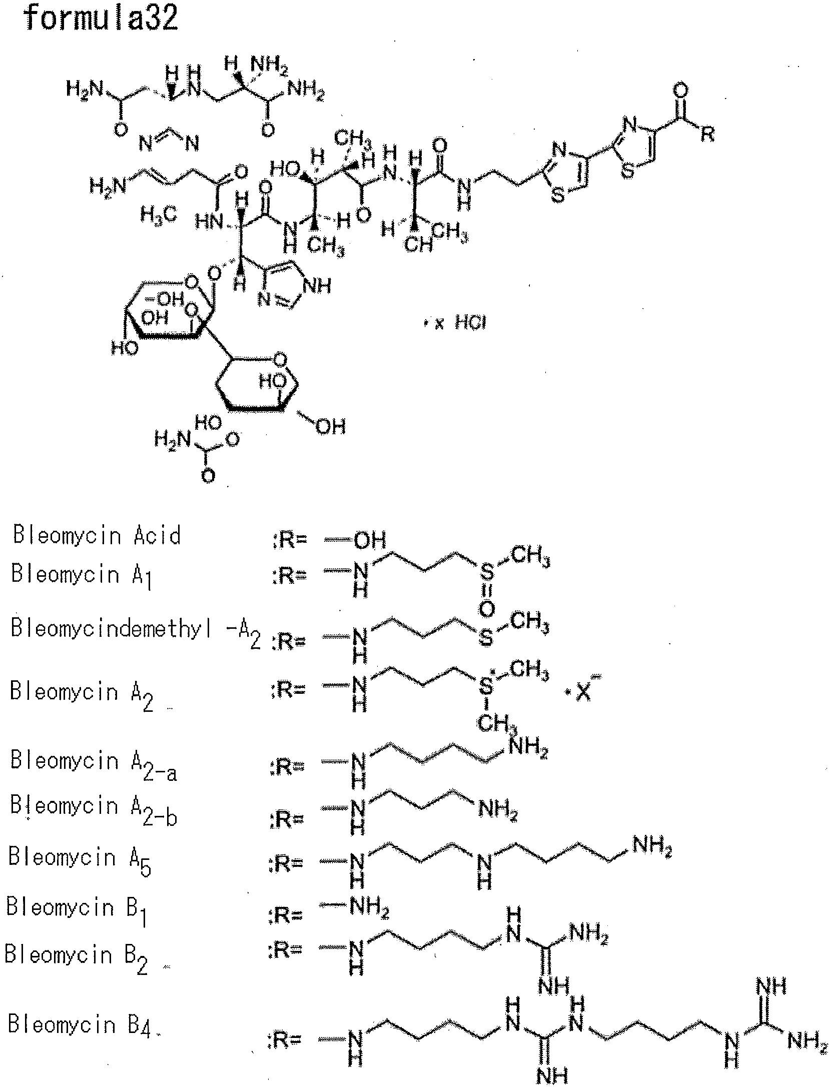
(76) general name: peplomycin sulfate, antitumor antibiotic 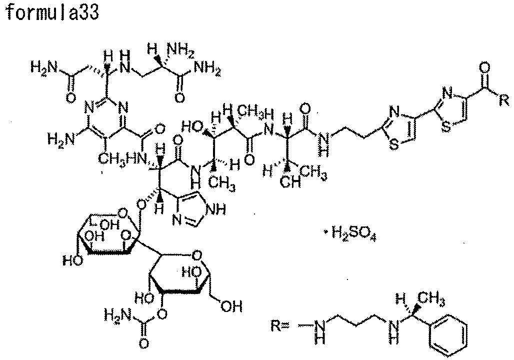
(77) general name: mitomycin C, antitumor antibiotic 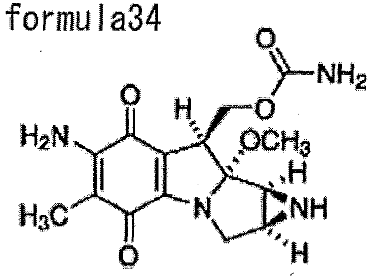
(78) general name: amrubicin hydrochloride, antitumor antibiotic 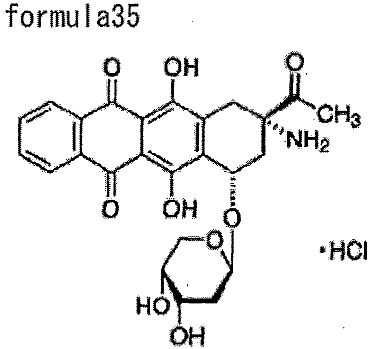
(79) general name: vibramycin hydrochloride, antitumor antibiotic 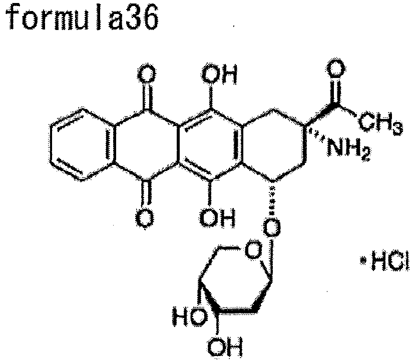
(80) general name: pirarubicin hydrochloride, antitumor antibiotic 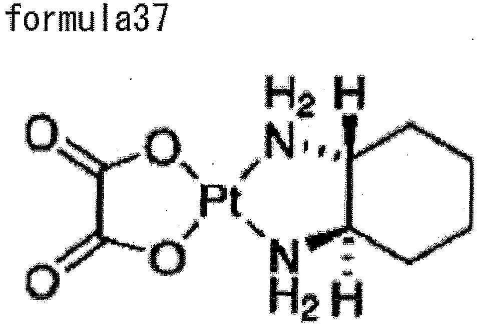
(81) general name: docetaxel hydrate, microtubule inhibitor 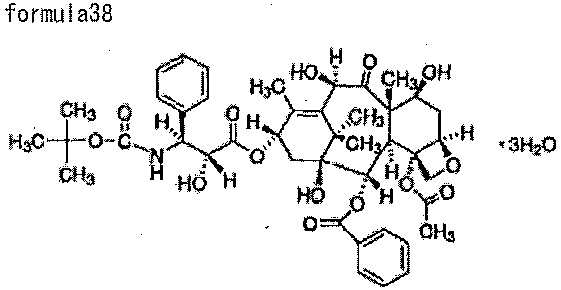
(82) general name: vincristine sulfate, microtubule inhibitor 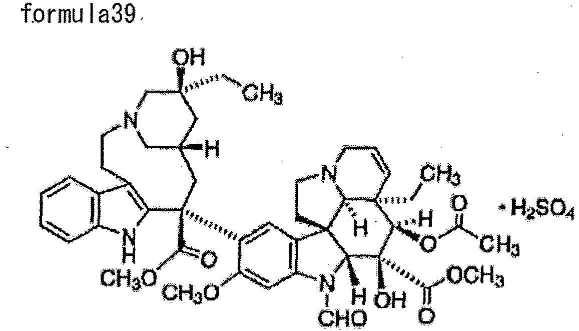
(83) general name: vinblastine sulfate, microtubule inhibitor 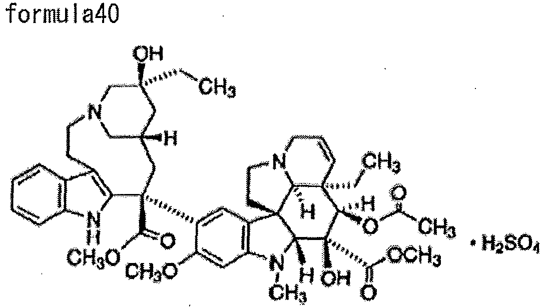
(84) general name: vinorelbine ditartrate, microtubule inhibitor 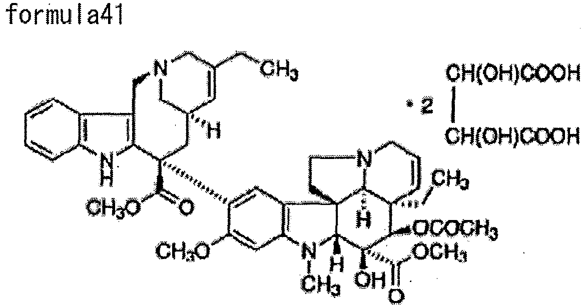
(85) general name: vindesine sulfate, microtubule inhibitor 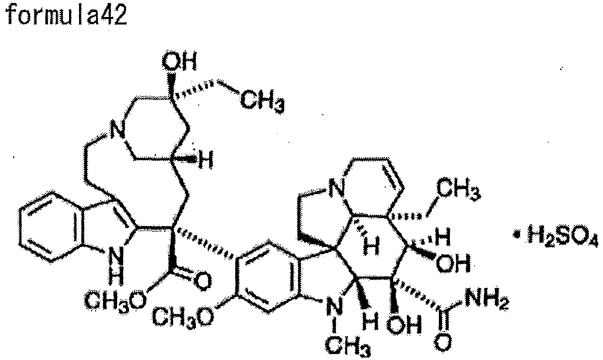
(86) general name: oxaliplatin, platinum preparation 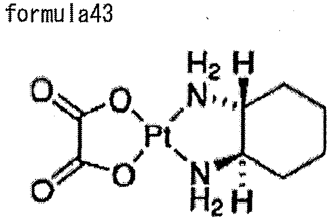
(87) general name: carboplatin, platinum preparation 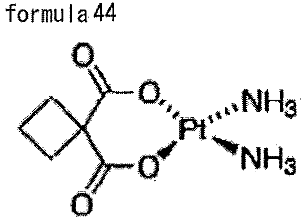
(88) general name: cisplatin, platinum preparation 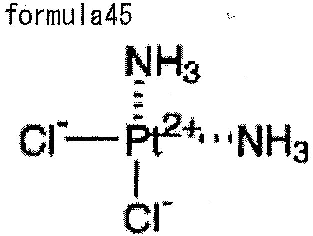
(89) general name: nedaplatin, platinum preparation 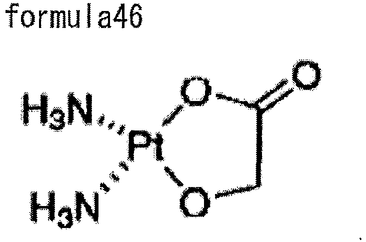
(90) general name: anastrozole, parahormone drug 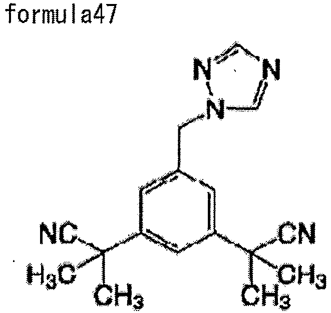
(91) general name: Afema, parahormone drug 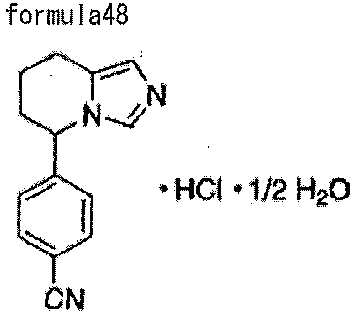
(92) general name: exemestane, parahormone drug 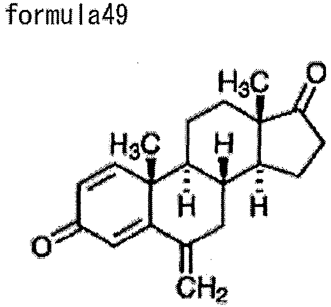
(93) general name: tamoxifen citrate, parahormone drug 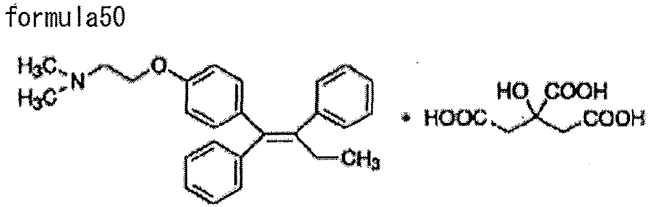
(94) general name: toremifene citrate, parahormone drug 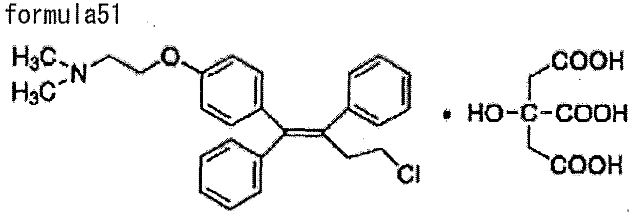
(95) general name: bicalutamide, parahormone drug 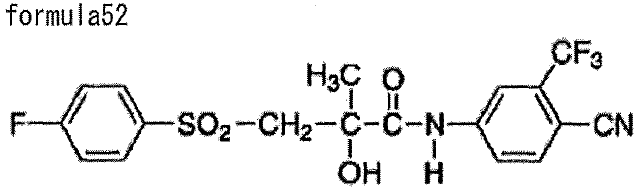
(96) general name: flutamide, parahormone drug 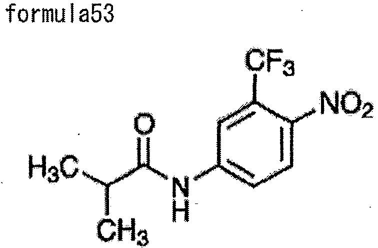
(97) general name: mepitiostane, parahormone drug 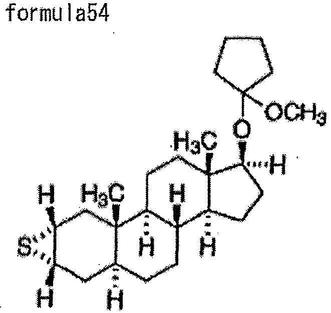
(98) general name: estramustine sodium phosphate, parahormone drug 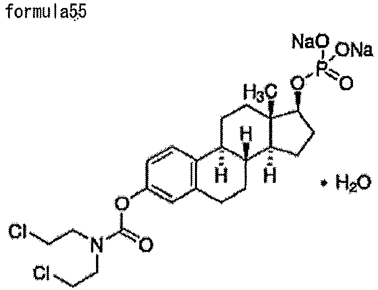
(99) general name: medroxyprogesterone acetate, parahormone drug 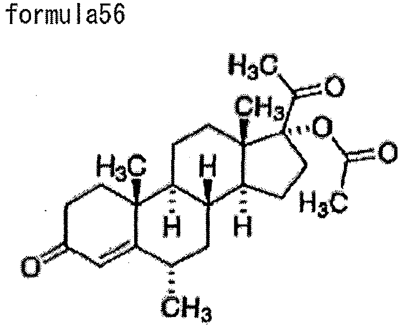
(100) general name: tamibarotene, molecular target drug 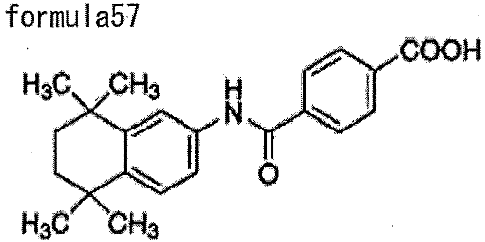
(101) general name: Gefitinib, molecular target drug 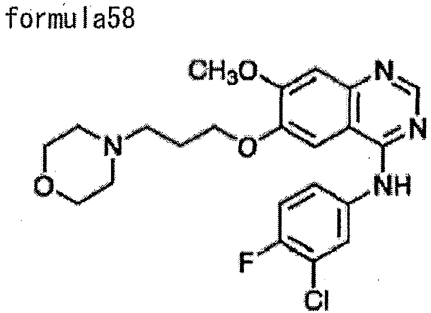
(102) general name: tretinoin, molecular target drug 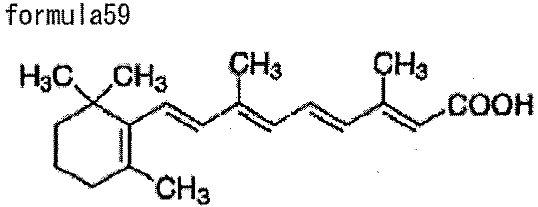
(103) general name: imatinib mesylate, molecular target drug 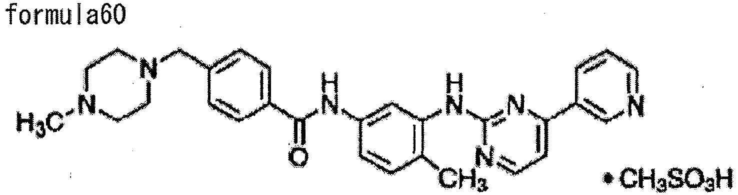
(104) general name: etoposide, topoisomerase inhibitor 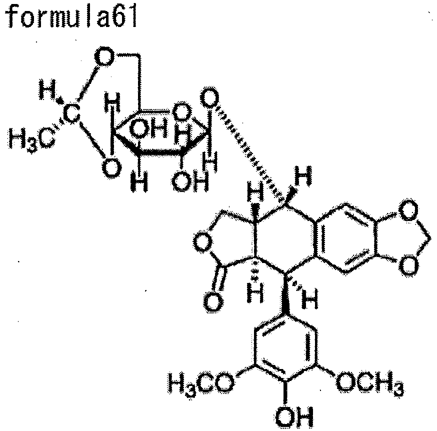
(105) general name: sobuzoxane, topoisomerase inhibitor 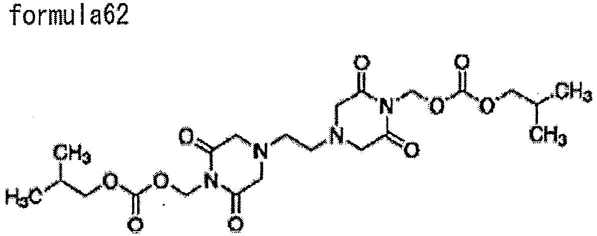
(106) general name: irinotecan hydrochloride, topoisomerase inhibitor 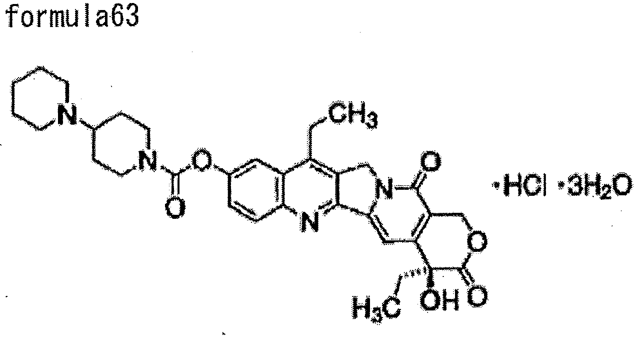
(107) general name: nogitecan hydrochloride, topoisomerase inhibitor 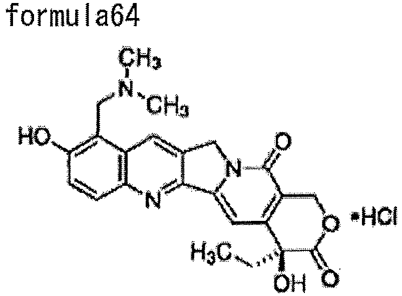
(108) general name: ubenimex, nonspecific immunopotentiator 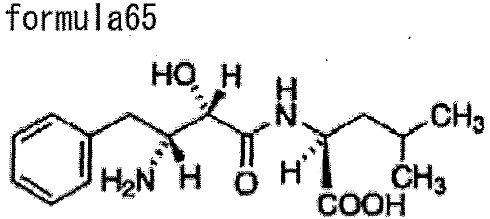
(109) general name: sizofiran, nonspecific immunopotentiator) 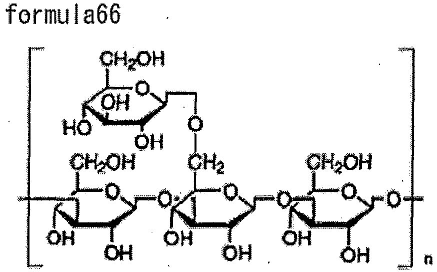
(110) general name: lenthinan, nonspecific immunopotentiator.

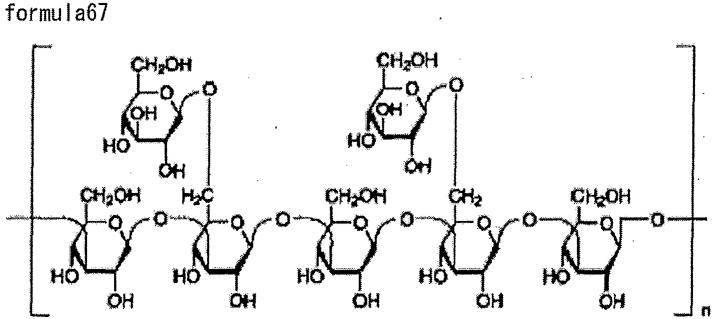

Furthermore, the present invention provides an antineoplastic drug having a self-magnetic metal-salen complex compound wherein R₃ is composed of any one of compounds represented by the following formulas (111) to (116):
(111) product name: Leuplin; and general name: leuprorelin acetate, anti-tumor agent 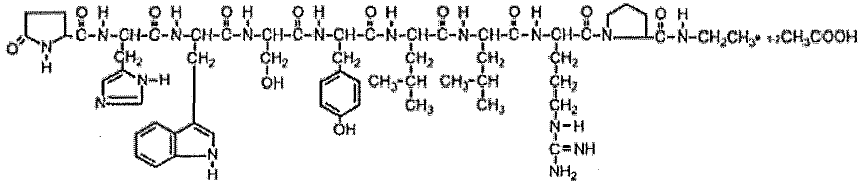
(112) product name: methotrexate; and general name: methotrexate, anti-tumor agent 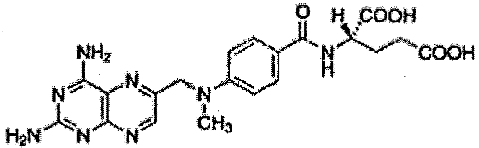
(113) product name: Novantrone; and general name: mitoxantrone hydrochloride, anti-tumor agent 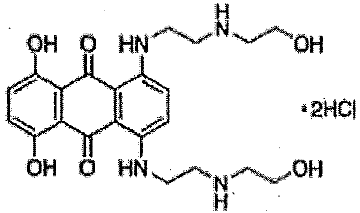
(114) product name: photofrin; and general name: porfimer sodium, anti-tumor agent 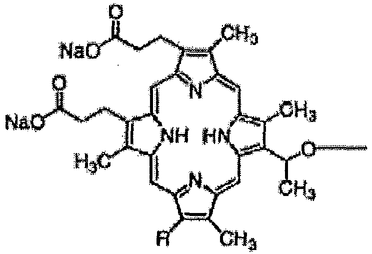
(115) product name: photofrin; and general name: porfimer sodium, anti-tumor agent 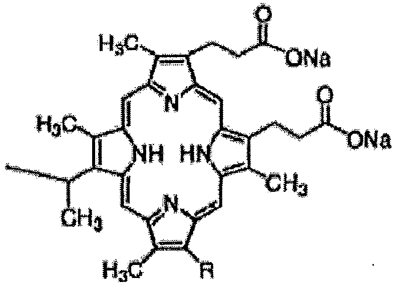
(116) product name: Mylotarg; and general name: gemtuzumab ozogamicin, anti-tumor agent.

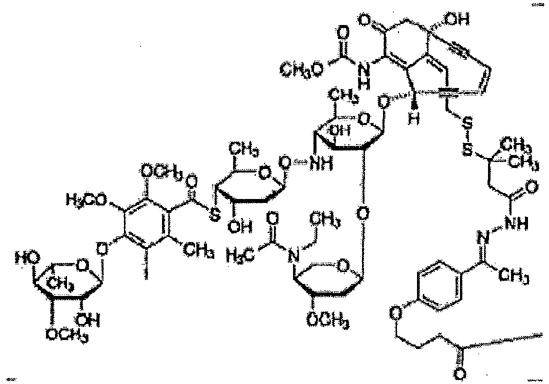

### [Advantageous Effects of Invention]

The structures of metal-salen complexes, which exerts pharmacological effects, and their derivatives are clarified according to the present invention.

### [Brief Description of Drawings]

[Fig. 1] Fig. 1 is a graph showing changes in weight (TG) and the results of differential thermal analysis (DTA) with respect to metal-salen complexes according to the present invention.
[Fig. 2] Fig. 2 is a diagram showing an integral curve of the results of mass spectrometry for the metal-salen complexes according to the present invention.
[Fig. 3] Fig. 3 is an enlarged view (Y axis direction) of Fig. 2.
[Fig. 4] Fig. 4 is a diagram showing a magnetic field-magnetization curve of a Mn salen complex.
[Fig. 5] Fig. 5 is a diagram showing a magnetic field-magnetization curve of a Cr salen complex.
[Fig. 6] Fig. 6 is a diagram showing a magnetic field-magnetization curve of a Co salen complex at 37 C°(310 K).
[Fig. 7] Fig. 7 shows a magnetic field-magnetization curve of a Fe salen complex.
[Fig. 8] Fig. 8 is a diagram showing magnetic field-magnetization curves of the Mn salen complex, Cr salen complex, and Fe salen complex.
[Fig. 9] Fig. 9 is a diagrammatic illustration of a state where a bar magnet is made to be in contact with a rectangular flask.
[Fig. 10] Fig. 10 is a characteristic diagram showing the relationship between the distance from the magnet and the number of cells per unit area.
[Fig. 11] Fig. 11 is a perspective view of a guidance system.
[Fig. 12] Fig. 12 is a characteristic diagram showing SNR measurement results of cells after being placed on the guidance system by using MRI.
[Fig. 13] Fig. 13 is photographs showing the effects of the Fe salen complex on melanoma growth in mice.
[Fig. 14] Fig. 14 is a characteristic diagram showing the effects of the Fe salen complex on melanomas.
[Fig. 15] Fig. 15 is a diagram showing the results of a histological examination of the Fe salen complex.
[Fig. 16] Fig. 16 is a graph showing the relationship between magnetic field intensity of the Fe salen complex and a temperature rise.

### [Description of Embodiments]

### (Example 1)

A metal-salen complex according to the present invention was produced in the following manner.

### Step 1:

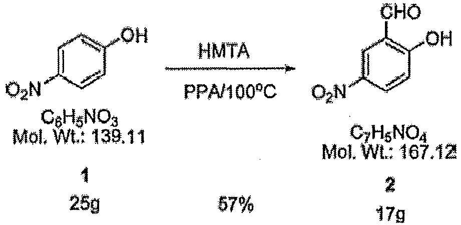

A mixture of 4-nitrophenol (25g, 0.18 mol), hexamethylene tetramine (25g, 0.18 mol), and polyphosphoric acid (200ml) were stirred for one hour at the temperature of 100 degrees Celsius. Then, that mixture was introduced to 500ml of ethyl acetate and 1 L (liter) of water and stirred until it completely dissolved. Furthermore, when 400 ml of ethyl acetate was added to that solution, the solution separated into two phases. Subsequently, the aqueous phase was removed from the solution which separated into the two phases; and the remaining compound was washed twice with a basic solvent and dried over anhydrous MgSO₄. As a result, 17 g of Compound 2 (57% yield) was synthesized.

### Step 2:

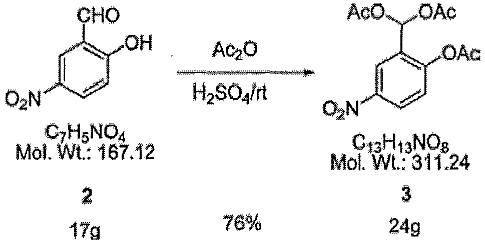

Compound 2 (17g, 0.10 mol), acetic anhydride (200 ml) and H₂SO₄ (minimal) were stirred for one hour at room temperature. The resulting solution was mixed for 0.5 hour in iced water (2 L) to bring about hydrolysis. The resulting solution was filtered and dried in air, thereby obtaining white powder. The powder was recrystallized, using a solvent containing ethyl acetate. As a result, 24 g of Compound 3 (76% yield) was obtained in the form of white crystals.

### Step 3:

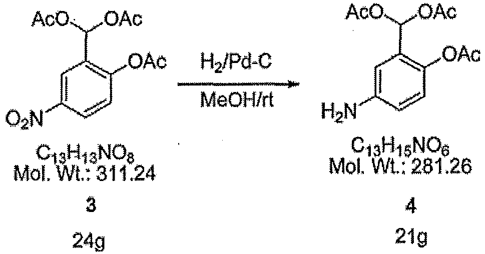

A mixture of carbon (2.4 g) supporting 10% palladium with Compound 3 (24 g, 77 mmol) and methanol (500 ml) was reduced over night in a 1.5 atm hydrogen reducing atmosphere. After the reduction was completed, the product was filtered, thereby allowing 21 g of Compound 4 in the form of brown oil to be synthesized.

### Steps 4 and 5:

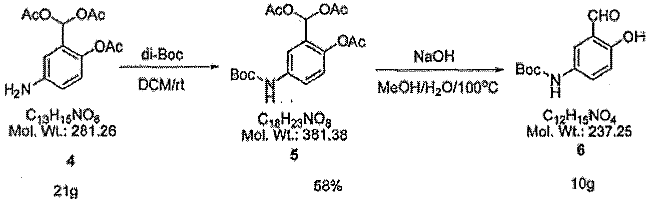

Compound 4 (21 g, 75 mmol) and di(tert-butyl)dicarbonate (18 g, 82 mmol) were stirred over night in anhydrous dichloromethane (DCM) (200 ml) in a nitrogen atmosphere. The resulting solution was allowed to evaporate in a vacuum and then dissolved in methanol (100 ml). Sodium hydroxide (15 g, 374 mmol) and water (50 ml) were then added and the solution was brought to reflux for 5 hours. The solution was then cooled, filtered, washed with water, and allowed to dry in a vacuum, thereby obtaining a brown compound. The resulting compound was processed twice by flash chromatography using silica gel, thereby obtaining 10 g of Compound 6 (58% yield).

### Step 6:

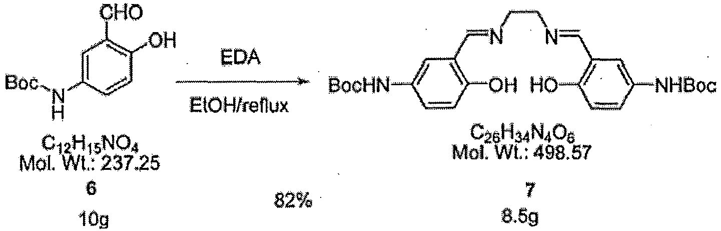

Compound 6 (10 g, 42 mmol) was introduced into 400 ml of anhydrous ethanol, the mixture was brought to reflux while heated, and several drops of ethylene diamine (1.3 g, 21 mmol) were added into 20 ml of anhydrous ethanol while stirred for 0.5 hour. The mixture was introduced into a container of ice, where it was cooled and mixed for 15 minutes. It was then washed with 200 ml of ethanol, filtered, and dried in a vacuum, thereby obtaining 8.5 g (82% yield) of Compound 7.

### Step 7:

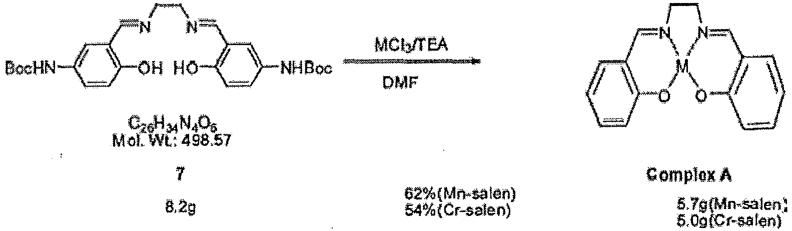

Compound 7 (8.2 g, 16 mmol) and triethylamine (22 ml, 160 mmol) were introduced into normal methanol (methanol made by Showa Chemical, purity 99.5% or more) (50 ml); and a solution of FeCl₃ · 4H₂O (2.7 g, 16 mmol) in a case of the Fe salen, MnCl₃ · 4H₂O (2.7 g, 16 mmol) in a case of the Mn salen, or CrCl₃ · 4H₂O (2.7 g, 16 mmol) in a case of the Cr salen added to 10 ml of methanol was mixed in a nitrogen atmosphere. Then the ingredients were mixed for one hour in a nitrogen atmosphere at the room temperature, thereby obtaining a brown compound. Subsequently, this compound was then dried in a vacuum or its water was dried sufficiently by, for example, using magnesium, or was adsorbed and removed by magnesium. The resulting compound was diluted with 400 ml of dichloromethane, washed twice with a basic solution, dried in Na₂SO₄, and dried in a vacuum, thereby obtaining a metal-salen complex compound of a dimer containing water molecules.

The resulting compound was recrystallized in a solution of diethyl ether and paraffin, and assay by high performance liquid chromatography revealed a metal-salen complex containing water molecules of purity of 95% or higher. The chemical structure formula of the obtained dimer with water molecules is as follows.

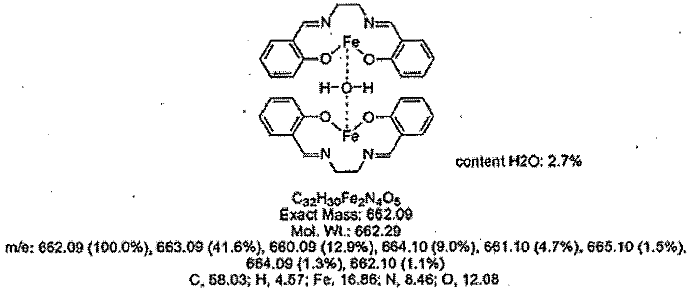

Incidentally, the bond between the metal and oxygen can be considered as a fusion of a covalent bond and a metallic bond.

Elemental analysis of the obtained dimer with water molecules revealed that it contained 57.73% C, 4.42% H, 17.2% Fe, 8.49% N, and 12.16% O; and all differences between calculated values and experimental values were within an absolute error range of ±0.4%.

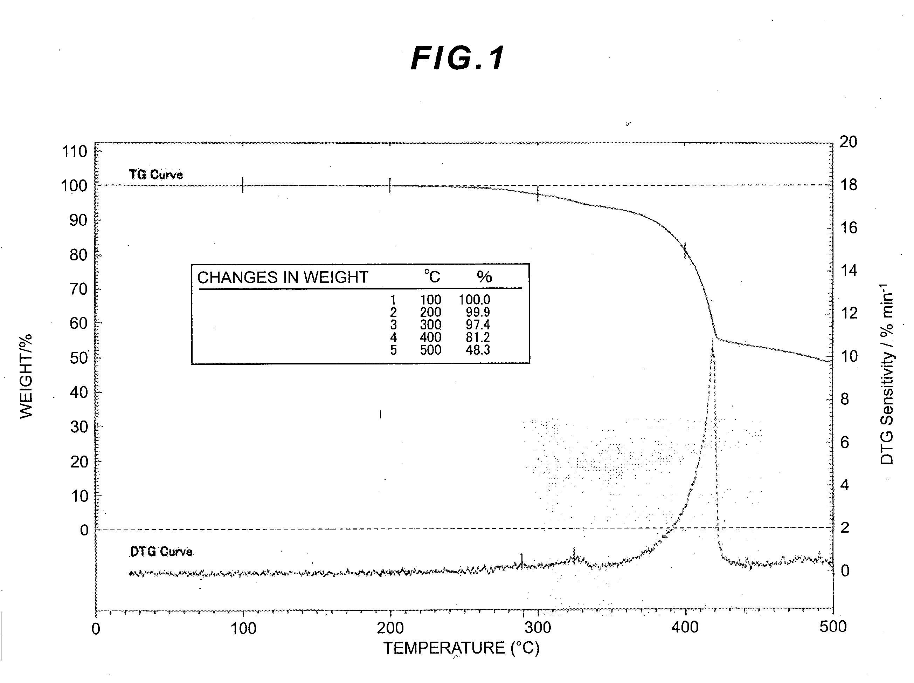

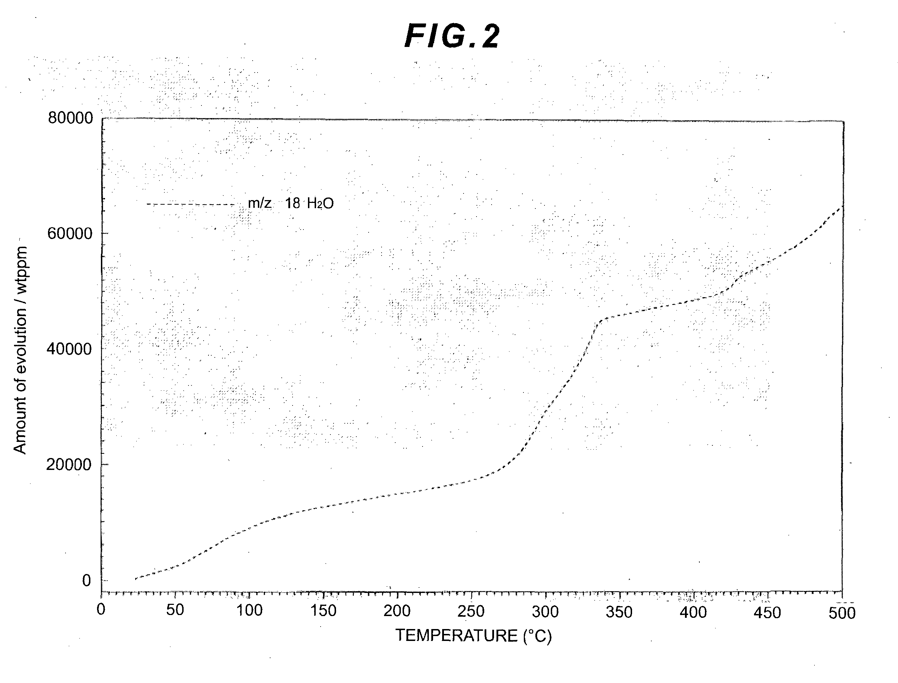

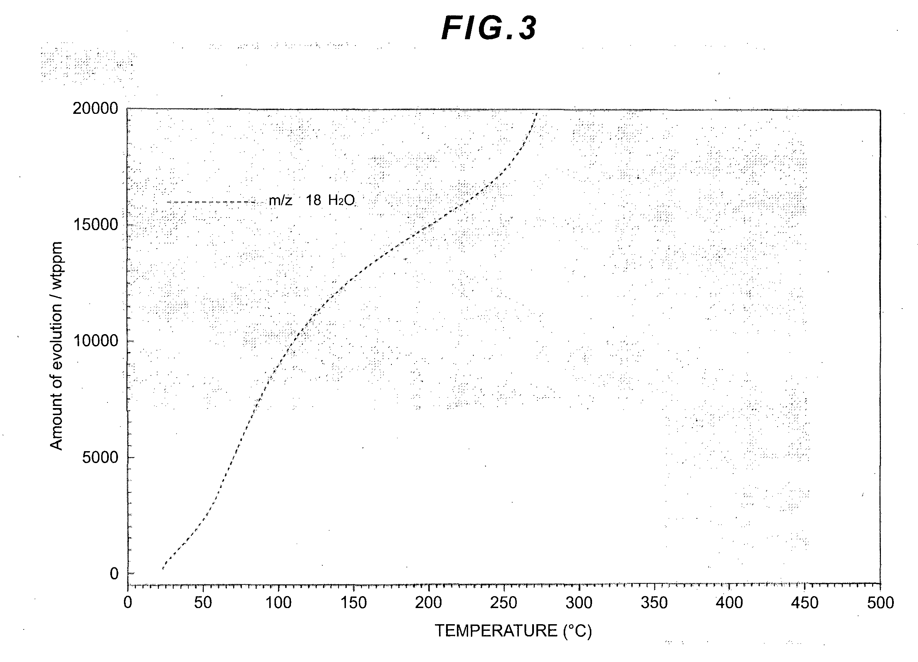

Next, TG-Mass analysis was performed in order to clarify the existence of the included water molecules. The results of the TG-Mass analysis are shown in Fig. 1 to Fig. 3. A TGA curve illustrated in Fig. 1 shows that the mass of a sample is 100% at room temperature at 100 degrees Celsius or lower and becomes 99.9% at 200 degrees Celsius and 97.4% at 300 degrees Celsius. Also, the results of the mass spectrometry illustrated in Fig. 2 and Fig. 3 show that most of the water molecules have been eliminated. Furthermore, the mass reduction at 300 degrees Celsius is 2.6%. This suggests that an amount of water molecules corresponding to one water molecule (mass: 2.7%) per two Fe salen molecules is incorporated into the crystals.

Incidentally, experimental conditions are as described below.
TG Device: TG-40 by SHIMADZU CORPORATION
MS Device: GC/MS QP2010(1) by SHIMADZU CORPORATION
Measurement Conditions
Before starting measurement: after setting the sample on the TG device, feed carrier gas for 15 minutes or more and then start increasing the temperature
Heating condition: from room temperature to 500 degrees Celsius (temperature rise speed: 5 degrees Celsius/min)
Sample Weight: 3.703 mg
MS Sensitivity: 1.80 kV
Mass Number Range: m/z=10-300
Atmosphere: helium (50ml/min)
Standard Reference Material: sodium tungstate dihydrate, 1-butene, carbon dioxide

### (Example 2)

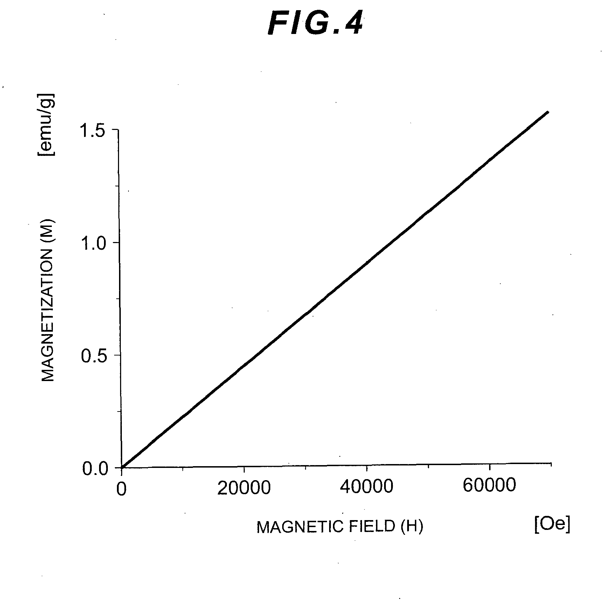

A magnetic field-magnetization curve of the Mn salen complex at 37 degrees Celsius (310 K) was measured by using MPMS7 by Quantum Design, Inc. and the measurement revealed that the Mn salen complex was paramagnetic. Fig. 4 shows the results.

### (Example 3)

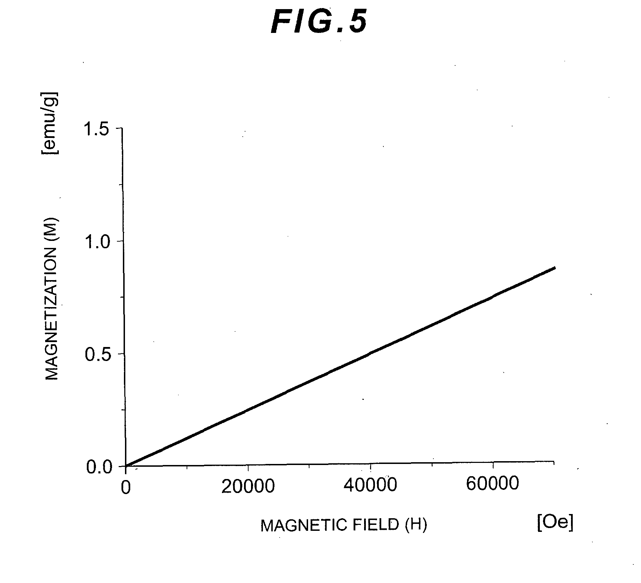

A magnetic field-magnetization curve of the Cr salen complex at 37 degrees Celsius (310 K) was measured by using MPMS7 by Quantum Design, Inc. and the measurement revealed that the Cr salen complex was paramagnetic. Fig. 5 shows the results.

### (Example 4)

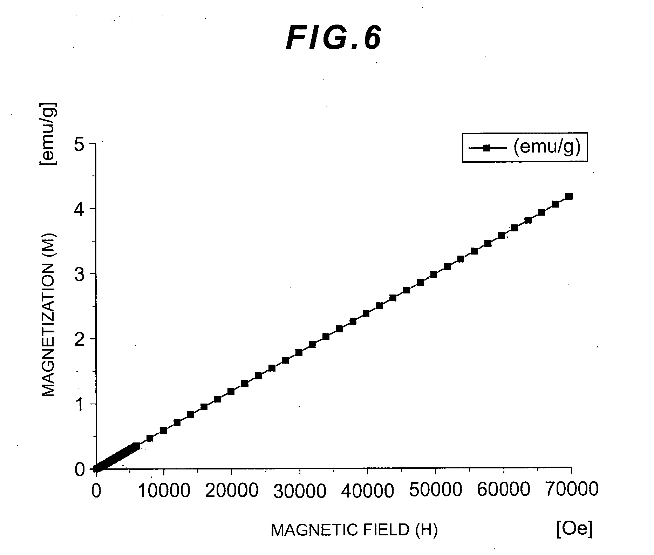

A magnetic field-magnetization curve of the Co salen complex at 37 degrees Celsius (310 K) was measured by using MPMS7 by Quantum Design, Inc. and the measurement revealed that the Co salen complex was paramagnetic. Fig. 6 shows the results.

### (Example 5)

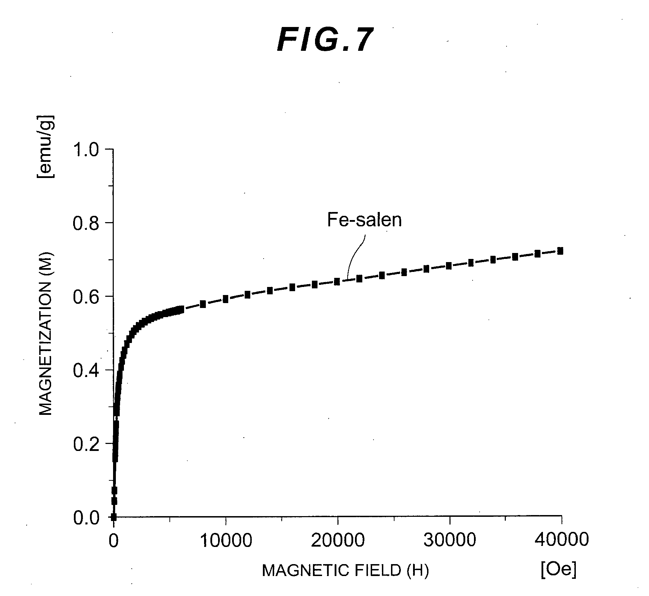

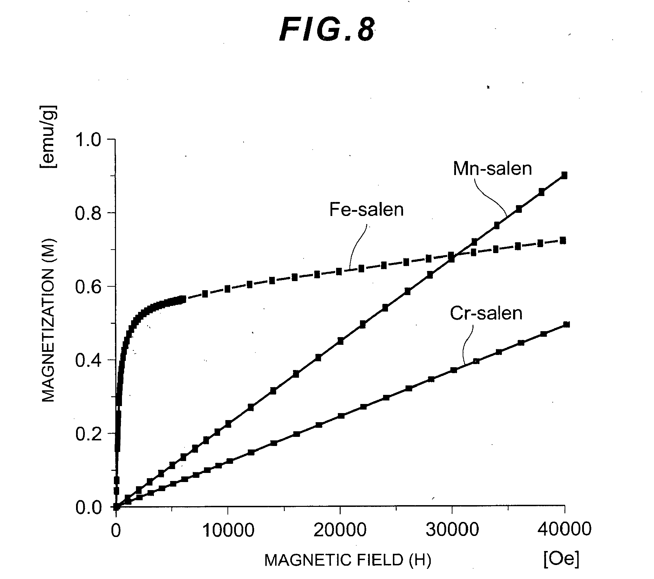

Fig. 7 shows a magnetic field-magnetization curve of the Fe salen complex at 37 degrees Celsius (310 K). Also, Fig. 8 is a graph showing the magnetic field-magnetization curves of the Mn salen complex, the Cr salen complex, and the Fe salen complex. Fig. 8 shows that: the Fe salen complex has larger magnetization than the Cr salen complex; and the Mn salen complex has larger magnetization than the Fe salen complex when the magnetic field is 30000 Oe (3T) or more. Therefore, the Fe salen complex has the largest magnetization when the magnetic field is less than 10000 Oe (1T); and is suited for use in magnetic induction drug delivery systems which use, for example, neodymium permanent magnets. However, when the magnetic field is 30000 Oe (1T) or more, the Mn salen complex has the largest magnetization and is most suited for magnetic induction drug delivery systems which use superconducting magnets.

### (Example 6)

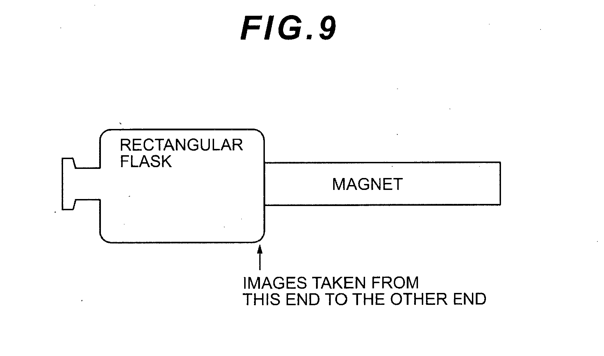

Culture medium was sprinkled with metal-salen complex powder, which is obtained with respect to each of the Fe salen complex, the Mn salen complex, the Cr salen complex, and the Co salen complex by the above-described method, in amounts allowing magnetic attraction to be visibly observed at a rat L6 cell confluence of 30%, and the state of the medium was photographed after 48 hours. Incidentally, FIG. 9 shows a bar magnet in contact with a rectangular flask containing rat L6 cell culture medium.

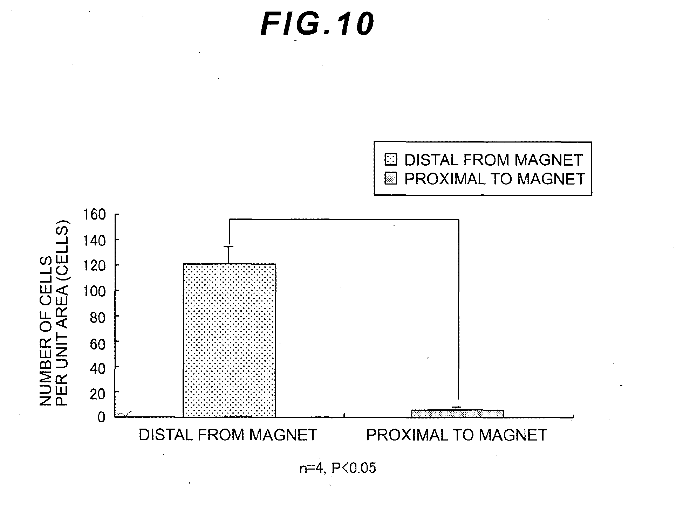

After 48 hours, the bottom face of the rectangular flask was photographed from one end to the other, and the cell count was calculated, with the results shown in FIG. 10. Referring to FIG. 10, a position proximal to the magnet means within a projection area of the magnet end surface at the bottom of the rectangular flask, and a position distal to the magnet means a region on the side opposite the magnet end surface at the bottom of the rectangular flask.

FIG. 10 shows that, near the magnet, the Mn salen complex was attracted, resulting in a greater Fe-salen complex concentration, so that the DNA-growth inhibition action of the Fe-salen complex resulted in a dramatically lower number of cells than the position distal to the magnet. As a result, the magnetic drugs and the system equipped with magnetism-generating means according to the present invention can thus allow the drugs to become concentrated in target tissues and affected sites of individuals.

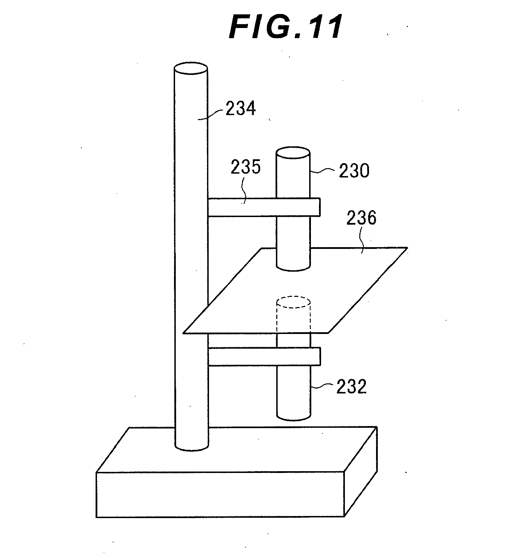

Next, another example of the delivery device of the invention will be described. In this delivery device, as illustrated in FIG. 11, a pair of magnets 230 and 232 facing each other in the direction of gravity are supported by a stand 234 and clamp 235, and a metal plate 236 is located between the magnets 230 and 232. The metal plate 236, especially an iron plate, is placed between the pair of magnets 230 and 232 so that a magnetic field of locally uniform and strong strength can be created.

An electrical magnet can be used instead of a magnet to modify the magnetic force generated in this delivery device. The magnetism-generating means can be moved to a target position of the individual on a table to allow the pair of magnetism-generating means to move in the X, Y, and Z directions. The tissue of an individual can be placed in the region of the magnetic field to concentrate the drug in the tissue.

More specifically, for example, the aforementioned metal complex (drug concentration: 5 mg/mL (15 mM)) was injected intravenously into a mouse weighing about 30 g, a laparotomy was performed, and the mouse was placed on the iron plate to locate its right kidney between the pair of magnets. Incidentally, the magnets used were Product No. N50 (neodymium permanent magnets) by Shin-Etsu Chemical Co., Ltd. with a residual flux density of 1.39 to 1.44 T. Under this circumstance, the magnetic field applied to the right kidney was about 0.3 (T), and the magnetic field applied to its left kidney was about 1/10 of the above-mentioned magnetic field.

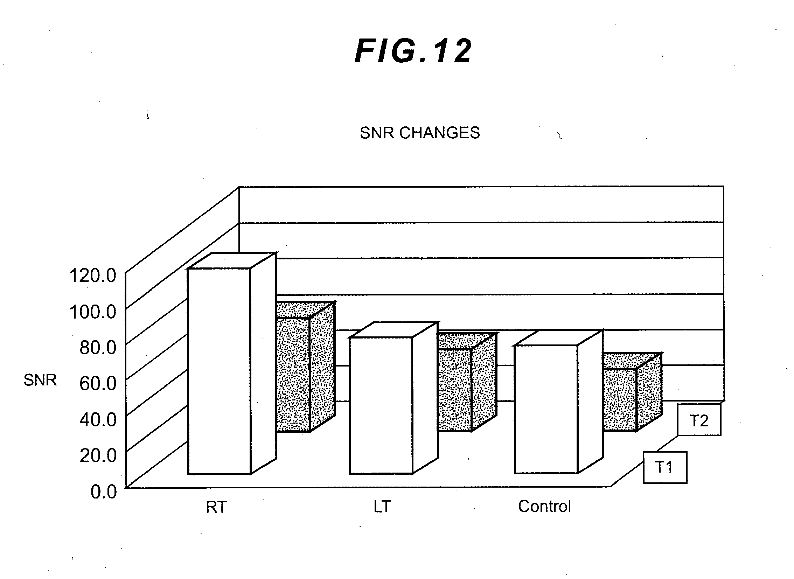

Together with the left kidney and a kidney to which no field was applied (control), a magnetic field was applied to the right kidney of the mouse; and after 10 minutes the SNR was measured by MRI in T1 mode and T2 mode. As shown in FIG. 12, it was confirmed that the drug stayed in the right kidney (RT) to which the magnetic field was applied, as compared to the left kidney (LT) and control.

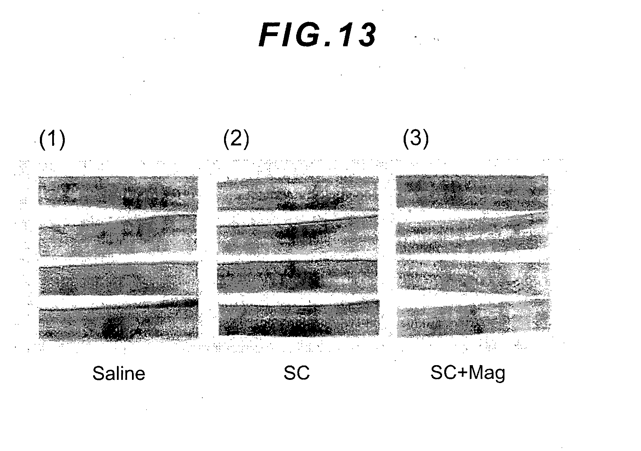

FIG. 13 shows the effect of the Fe salen complex on melanoma growth in mice. Melanoma was established in mouse tails in vivo by local grafting of cultured melanoma cells (Clone M3 melanoma cells). Incidentally, Fig. 13(1) is a photograph showing the effects of a saline group (saline) for which the saline water was injected instead of the Fe salen complex; Fig. 13(2) is a photograph showing the effects of a group (SC) for which the Fe salen complex was injected without applying the magnetic field; and Fig. 13(3) is a photograph showing the effect of a group (SC + Mag) for which the Fe salen complex was injected while applying the magnetic field (n=7-10).

The Fe salen complex (50 mg/kg) was administered intravenously via tail vein, followed by local application of a magnetic field by the use of a commercially available bar magnet (630 mT, a cylindrical neodymium magnet, 150 mm long and 20 mm in diameter). Application of a bar magnet was performed with 3 hours gentle contact with the site of melanoma immediately after injection of the salen complex for 10-14 days.

Application of the bar magnet was performed in such a way so that the magnetic field strength became maximal over the area of expected melanoma extension, which was approximately 150 mm or shorter in a mouse tail with the growth period of 2 weeks. Twelve days after the initial injection of the Fe salen complex, the extension of melanoma was evaluated by assessing the area of melanoma pigmentation.

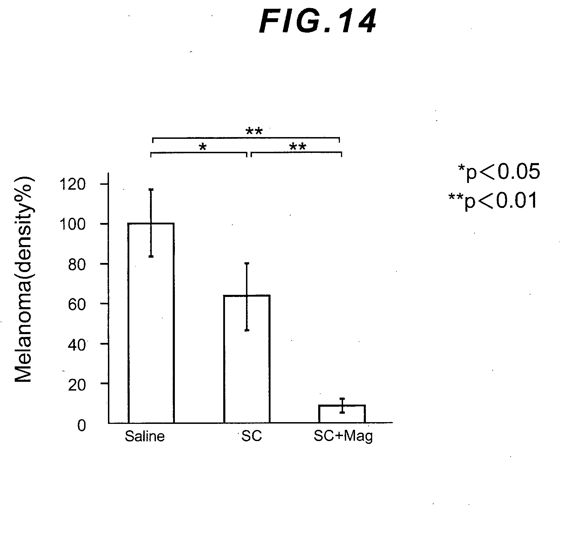

As shown in FIG. 14, the melanoma extension was greatest in the saline group (100±17.2%), in which saline, instead of the Fe salen complex, was injected, while the melanoma extension modestly decreased (63.68±16.3%) in the SC group, in which the Fe salen complex was injected without the application of a magnetic force field. In contrast, most melanoma disappeared (9.05±3.42%) in the SC+Mag group, in which the Fe salen complex was injected and a magnet force field was applied as described above (n=7-10).

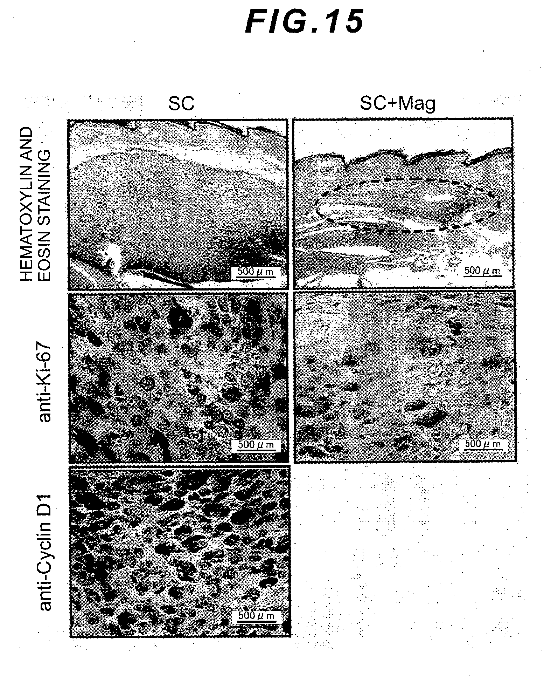

A histological examination was performed as shown in Fig. 15 by means of Hematoxylin-Eosin staining and immuno-histochemical staining with an anti-Ki-67 antibody and an anti-Cyclyn D1 antibody, which are both tumor proliferation markers, in tissue sections. As a result, the histological examination revealed that tumor expansion of melanoma diminished when the Fe salen complex was injected (SC); and the tumor expansion of melanoma mostly disappeared when the magnetic force field application was combined with the Fe salen complex.

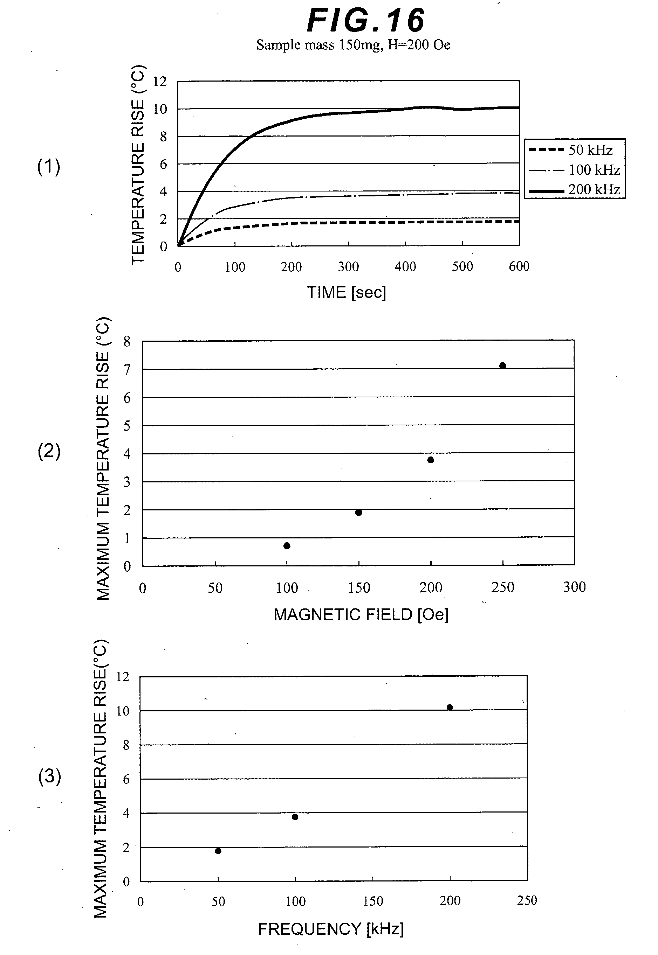

Furthermore, the application of an AC magnetic field with a magnetic field intensity of 200 Oe and a frequency of 50 kHz to 200 kHz to the drug (Fe salen complex; 9.25 mmol) increased the drug temperature from 2 degrees Celsius to 10 degrees Celsius (FIG. 16). This confirmed that the temperature zone allowed cancer cells to be killed from 39 degrees Celsius to 47 degrees Celsius as calculated in terms of temperature during administration to the living body. Incidentally, Fig. 16(1) shows changes in temperatures with time when the AC magnetic field was applied to the drug; Fig. 16(2) shows the maximum temperature when only the magnetic field was changed while using a fixed frequency; and Fig. 16(3) shows the maximum temperature when only the frequency was changed while using a fixed magnetic field.

### (Example 7)

The electron transfer of a compound which binds with the metal-salen complexes can be determined by first principles calculation. A system for realizing this computer simulation is equipped with well-known hardware resources as a computer, that is, memory, a computing device equipped with computing circuitry such as a CPU, and display means for outputting the computed results.

The memory includes data specifying existing organic compounds or three-dimensional structures, and software programs for performing computer simulation. The software program is capable of adding, modifying, and deleting compound side chains, cross linking certain side chains, calculating areas of high spin charge density, and determining the spin charge density for structures as a whole. For example, a commercially available program (Dmol3 by Accelrys) can be used as this program.

The user inputs the position where the side chains are to be added to a compound or selects one in which the side chains are modified or deleted, and uses a memory assisting program to designate on the computer the location where cross linking should be formed. The computer receives the input values to calculate the spin charge density, and outputs the results on a display screen. The user can also add structural data on existing compounds to the computer system to obtain the spin charge density of existing compounds.

The charge transfer of another compound binding to the metal salen complex can be determined by integrating the previously determined upward and downward spin charge density in three-dimensional space. The calculated results for charge transfer to e, b, k, h, or e, h of the aforementioned chemical formula (I) are given in each of the following tables. With each table, a minus sign (-) indicates an increase of electrons and a plus sign (+) indicates a decrease of electrons.
[Table 1]

**Table 1**

| Metal Salen Complex (Chemical Formula I) | Compound To Be Combined | |
|---|---|---|
| Charge Transfer | Compound Name | Charge Transfer |
| -0.31 | Ibuprofen chemical formula (1) | +0.31 |
| -0.31 | Mefenamic acid chemical formula (2) | +0.31 |
| -0.32 | Pefloxacin chemical formula (3) | +0.32 |
| -0.31 | Gemfibrozil chemical formula (4) | +0.31 |
| -0.32 | Rhodamine chemical formula (5) | +0.32 |
| -0.35 | Estrogen chemical formula (6) | +0.35 |
| -0.35 | Estrogen chemical formula (7) | +0.35 |
| -0.34 | Taxol chemical formula (8) | +0.34 |
| -0.28 | Glycine chemical formula (9) | +0.28 |
| -0.28 | Alanine chemical formula (10) | +0.28 |
| -0.27 | Arginine chemical formula (11) | +0.27 |
| -0.27 | Asparagine chemical formula (12) | +0.27 |
| -0.25 | Asparatic acid chemical formula (13) | +0.25 |
| -0.26 | Cysteine chemical formula (114) | +0.26 |
| -0.26 | Glutamic acid chemical formula (15) | +0.26 |
| -0.25 | Histidine chemical formula (16) | +0.25 |
| -0.27 | Isoleucine chemical formula (17) | +0.27 |
| -0.26 | Leucine chemical formula (18) | +0.26 |
| -0.24 | Lysine chemical formula (19) | +0.24 |
| -0.28 | Methionine chemical formula (20) | +0.28 |
| -0.29 | Phenylalanine chemical formula (21) | +0.29 |
| -0.26 | Proline chemical formula (22) | +0.26 |
| -0.26 | Serine chemical formula (23) | +0.26 |
| -0.25 | Threonine chemical formula (24) | +0.25 |
| -0.28 | Tryptophan chemical formula (25) | +0.28 |
| -0.29 | Tyrosine chemical formula (26) | +0.29 |
| -0.25 | Valine chemical formula (27) | +0.25 |

[Table 2]

**Table 2**

| Metal Salen Complex (Chemical Formula I) | Compound To Be Combined | |
|---|---|---|
| Charge Transfer | Compound Name | Charge Transfer |
| -0.33 | Ifosfamide | +0.33 |
| -0.34 | Cyclophosphamide | +0.34 |
| -0.32 | Dacarbazine | +0.32 |
| -0.33 | Busulfan | +0.33 |
| -0.33 | Melphalan | +0.33 |
| -0.28 | Ranimustine | +0.28 |
| -0.30 | Estramustine sodium phosphate | +0.30 |
| -0.31 | Nimustine hydrochloride | +0.31 |
| -0.39 | Docetaxel hyderate | +0.39 |
| -0.38 | Vincristine sulfate | +0.38 |
| -0.38 | Vinblastine sulfate | +0.38 |
| -0.23 | Epirubicin hydrochloride | +0.23 |
| -0.33 | Vinorelbine ditartrate | +0.33 |
| -0.29 | Vindesine sulfate | +0.29 |
| -0.25 | Oxaliplatin | +0.25 |
| -0.22 | Carboplatin | +0.22 |
| -0.23 | Cisplatin | +0.23 |
| -0.24 | Nedaplatin | +0.24 |

[Table 3]

**Table 3**

| Metal Salen Complex (Chemical Formula I) | Compound To Be Combined | |
|---|---|---|
| Charge Transfer | Compound Name | Charge Transfer |
| -0.19 | Anastrozole | +0.19 |
| -0.18 | Afema | +0.18 |
| -0.28 | Exemestane | +0.28 |
| -0.13 | Toremifene citrate | +0.13 |
| -0.23 | Bicalutamide | +0.23 |
| -0.39 | Flutamide | +0.39 |
| -0.22 | Mepiotiostane | +0.22 |
| -0.30 | Estramustine sodium phosphate | +0.30 |
| -0.31 | Medroxyprogesterone acetate | +0.31 |
| -0.23 | Tamibarotene | +0.23 |
| -0.22 | Gefitinib | +0.22 |
| -0.24 | Tretinoin | +0.24 |
| -0.27 | Imatinib mesylate | +0.27 |
| -0.27 | Etoposide | +0.27 |
| -0.25 | Sobuzoxane | +0.25 |
| -0.22 | Irinotecan hydrochloride | +0.22 |
| -0.23 | Nogitecan hydrochloride | +0.23 |

[Table 4]

**Table 4**

| Metal Salen Complex (Chemical Formula I) | Compound To Be Combined | |
|---|---|---|
| Charge Transfer | Compound Name | Charge Transfer |
| -0.33 | Ubenimex | +0.33 |
| -0.31 | Sizofiran | +0.31 |
| -0.28 | Lenthinan | +0.28 |
| -0.33 | Ifosfamide | +0.33 |
| -0.34 | Cyclophosphamide | +0.34 |
| -0.32 | Dacarbazine | +0.32 |
| -0.33 | Busulfan | +0.33 |
| -0.33 | Melphalan | +0.33 |
| -0.28 | Ranimusutine | +0.28 |
| -0.30 | Estramustine sodium phosphate | +0.30 |
| -0.31 | Nimustine hydrochloride | +0.31 |

[Table 5]

**Table 5**

| Metal Salen Complex (Chemical Formula I) | Compound To Be Combined | |
|---|---|---|
| Charge Transfer | Compound Name | Charge Transfer |
| -0.23 | Enocitabine chemical Formula(3) | +0.23 |
| -0.24 | Capecitabine chemical formula(4) | +0.24 |
| -0.22 | Carmofur chemical formula(5) | +0.22 |
| -0.23 | Gimeracil chemical formula(6) | +0.23 |
| -0.33 | Oteracil potassium chemical formula(7) | +0.33 |
| -0.28 | Cytarabine chemical formula(8) | +0.28 |
| -0.30 | Cytarabine ocfosfate chemical formula(9) | +0.30 |
| -0.31 | Tegafur chemical formula(10) | +0.31 |
| -0.30 | Doxifluridine chemical formula(11) | +0.30 |
| -0.32 | Hydroxycarbamide chemical formula(12) | +0.32 |
| -0.33 | Fluorouracil chemical formula(13) | +0.33 |
| -0.35 | Mercaptopurine hydrate chemical formula(14) | +0.35 |
| -0.33 | Fludarabine phosphate chemical formula (15) | +0.33 |
| -0.34 | Gemcitabine hydrochloride chemical formula(16) | +0.34 |
| -0.33 | Actinomycin-D | +0.33 |
| -0.24 | Aclarubicin hydrochloride | +0.24 |
| -0.32 | Idarubicin hydrochloride | +0.32 |
| -0.23 | Epirubicin hydrochloride | +0.23 |
| -0.33 | Zinostatin stimalamer | +0.33 |
| -0.29 | Daunorubicin hydrochloride | +0.29 |
| -0.30 | Doxorubicin hydrochloride | +0.30 |
| -0.31 | Bleomycin hydrochloride | +0.31 |
| -0.19 | Peplomycin hydrochloride | +0.19 |
| -0.30 | Mitomycin C | +0.30 |
| -0.32 | Amrubicin hydrochloride | +0.32 |
| -0.33 | Pirarubicin hydrochloride | +0.33 |

[Table 6]

**Table 6**

| Metal Salen Complex (Formula II) | Compound To Be Combined | |
|---|---|---|
| Charge Transfer | Compound Name | Charge Transfer |
| -0.23 | Enocitabine | +0.23 |
| -0.24 | Capecitabine | +0.24 |
| -0.22 | Carmofur | +0.22 |
| -0.23 | Gimeracil | +0.23 |
| -0.33 | Oteracil potassium | +0.33 |
| -0.28 | Cytarabine | +0.28 |
| -0.30 | Cytarabine ocfosfate | +0.30 |
| -0.31 | Tegafur | +0.31 |
| -0.30 | Doxifluridine | +0.30 |
| -0.32 | Hydroxycarbamide | +0.32 |
| -0.33 | Fluorouracil | +0.33 |
| -0.35 | Mercaptopurine hydrate | +0.35 |
| -0.33 | Fludarabine phosphate | +0.33 |
| -0.34 | Gemcitabine hydrochloride | +0.34 |
| -0.33 | Actinomycin-D | +0.33 |
| -0.24 | Aclarubicin hydrochloride | +0.24 |
| -0.32 | Idarubicin hydrochloride | +0.32 |
| -0.23 | Epirubicin hydrochloride | +0.23 |
| -0.33 | Zinostatin stimalamer | +0.33 |
| -0.29 | Daunorubicin hydrochloride | +0.29 |
| -0.30 | Doxorubicin hydrochloride | +0.30 |
| -0.31 | Bleomycin hydrochloride | +0.31 |
| -0.19 | Peplomycin hydrochloride | +0.19 |
| -0.30 | Mitomycin C | +0.30 |
| -0.32 | Amrubicin hydrochloride | +0.32 |
| -0.33 | pirarubicin hydrochloride | +0.33 |

[Table7]

**Table 7**

| Metal Salen Complex (Chemical Formula II) | Compound To Be Combined | |
|---|---|---|
| Charge Transfer | Compound Name | Charge Transfer |
| -0.39 | Docetaxel hyderate | +0.39 |
| -0.38 | Vincristine sulfate | +0.38 |
| -0.38 | Vinblastine sulfate | +0.38 |
| -0.23 | Epirubicin hydrochloride | +0.23 |
| -0.33 | Vinorelbine ditartrate | +0.33 |
| -0.29 | Vindesine sulfate | +0.29 |
| -0.25 | Oxaliplatin | +0.25 |
| -0.22 | Carboplatin | +0.22 |
| -0.23 | Cisplatin | +0.23 |
| -0.24 | Nedaplatin | +0.24 |
| -0.19 | Anastrozole | +0.19 |
| -0.18 | Afema | +0.18 |
| -0.28 | Exemestane | +0.28 |
| -0.13 | Toremifene citrate | +0.13 |
| -0.23 | bicalutamide | +0.23 |
| -0.39 | Flutamide | +0.39 |
| -0.22 | Mepiotiostane | +0.22 |
| -0.30 | Estramustine sodium phosphate | +0.30 |
| -0.31 | Medroxyprogesterone acetate | +0.31 |

[Table8]

**Table 8**

| Metal Salen Complex (Chemical Formula II) | Compound To Be Combined | |
|---|---|---|
| Charge Transfer | Compound Name | Charge Transfer |
| -0.23 | Tamibarotene | +0.23 |
| -0.22 | Gefitinib | +0.22 |
| -0.24 | Tretinoin | +0.24 |
| -0.27 | Imatinib mesylate | +0.27 |
| -0.27 | Etoposide | +0.27 |
| -0.25 | Sobuzoxane | +0.25 |
| -0.22 | Irinotecan hydrochloride | +0.22 |
| -0.23 | Nogitecan hydrochloride | +0.23 |
| -0.33 | ubenimex | +0.33 |
| -0.31 | Sizofiran | +0.31 |
| -0.28 | Lenthinan | +0.28 |

[Table9]

**Table 9**

| Metal Salen Complex (Chemical Formula I) | Compound To Be Combined | |
|---|---|---|
| Charge Transfer | Compound Name | Charge Transfer |
| -0.22 | Lidocaine | +0.22 |
| -0.25 | Ethyl aminobenzoic acid | +0.25 |
| -0.25 | Oxybuprocaine | +0.25 |
| -0.24 | Oxethazaine | +0.24 |
| -0.23 | Dibucaine | +0.23 |
| -0.28 | Ethyl piperidinoacetylaminobenzoate | +0.28 |
| -0.25 | Procaine | +0.25 |
| -0.23 | Mepivacaine | +0.23 |
| -0.24 | p-butylaminobenzoyldiethylaminoethyl hydrochloride | +0.24 |
| -0.26 | Bupivacaine hydrochloride | +0.26 |
| -0.24 | Ropivacaine hydrochloride hydrate | +0.24 |
| -0.12 | Lidocaine | +0.12 |
| -0.15 | Ethyl aminobenzoic acid | +0.15 |
| -0.15 | Oxybuprocaine | +0.15 |
| -0.14 | Oxethazaine | +0.14 |
| -0.13 | Dibucaine | +0.13 |
| -0.18 | Ethyl piperidinoacetylaminobenzoate | +0.18 |
| -0.15 | Procaine | +0.15 |
| -0.13 | Mepivacaine | +0.13 |
| -0.14 | p-butylaminobenzoyldiethylaminoethyl hydrochloride | +0.14 |
| -0.16 | Bupivacaine hydrochloride | +0.16 |
| -0.14 | Ropivacaine hydrochloride hydrate | +0.14 |

[Table10]

**Table 10**

| Metal Salen Complex (Chemical Formula I) | Compound To Be Combined | |
|---|---|---|
| Charge Transfer | Compound Name | Charge Transfer |
| -0.32 | Leuplin chemical formula(3) | +0.32 |
| -0.35 | Methotrexate chemical formula(4) | +0.35 |
| -0.35 | Novanthrone chemical formula(5) | +0.35 |
| -0.34 | Photofrin chemical formula(6) | +0.34 |
| -0.33 | Photofrin chemical formula(7) | +0.33 |
| -0.28 | mylotarg | +0.28 |

## Claims

1. A metal-salen complex compound wherein a metal atom part in each of multiple molecules of a metal-salen complex or a derivative of the metal-salen complex is multimerized via water.

2. The metal-salen complex compound according to claim 1, containing the metal-salen complex or a dimer of the metal-salen complex.

3. The metal-salen complex compound according to claim 1 or 2, wherein the multimerized metal-salen complex or derivative of the metal-salen complex is self-magnetic.

4. A self-magnetic metal-salen complex compound represented by the following formula (I) 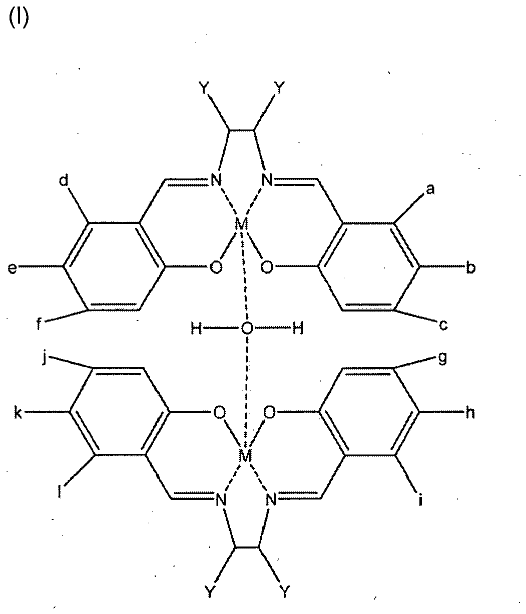 where M represents Fe, Cr, Mn, Co, Ni, Mo, Ru, Rh, Pd, W, Re, Os, Ir, Pt, Nd, Sm, Eu, or Gd, and each of a to f and Y is hydrogen or any one of the following (1) to (7):
(1) -CO₂Me;
(2) -CO(OCH₂CH₂)₂OCH₃;
(3) 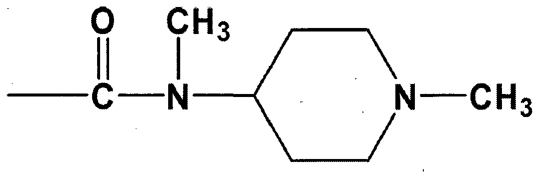
(4) 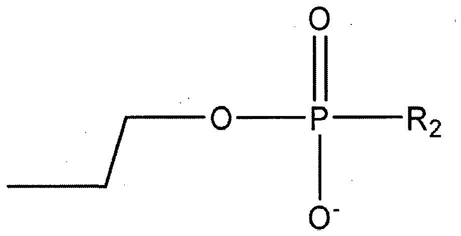 where R₂ represents a plurality of nucleic acids which are combined together and are formed of adenine, guanine, thymine, cytosine, or uracil;
(5) -NHCOH, -NH₂, -NHR₁, or -NR₁R₂
where R₁ and R₂ are alkyl or alkane with the same carbon number or the carbon number from 1 to 6;
(6) -NHR₃-, -NHCOR₃, or -R₃
where R₃ represents a substituent bound as a result of desorption of hydrogen or a hydroxyl group or a functional group such as a hydroxyl group or the like; and
(7) halogen atoms.

5. The self-magnetic metal-salen complex compound according to claim 4, wherein charge transfer of R₃ is less than 0.5 electrons.

6. The self-magnetic metal-salen complex compound according to claim 4 or 5, wherein R₃ is any one of compounds represented by the following formulas (8) to (34):
(8) ibuprofen piconol, phenylpropionic acid analgesic/anti-inflammatory 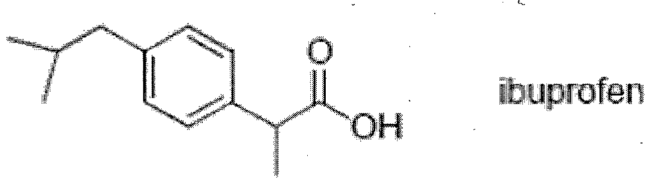
(9) mefenamic, anthranilic-acid anti-inflammatory analgesic 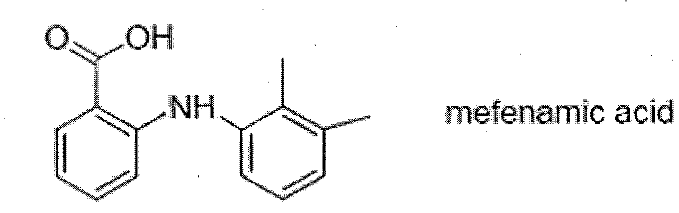
(10) drug for treating hyperlipemia 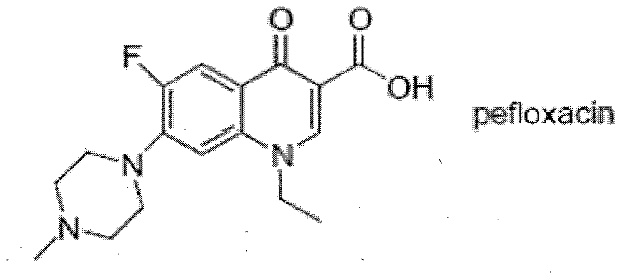
(11) antibacterial 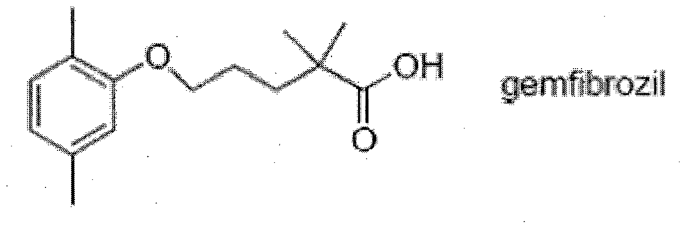
(12) fluorochrome (rhodamine) 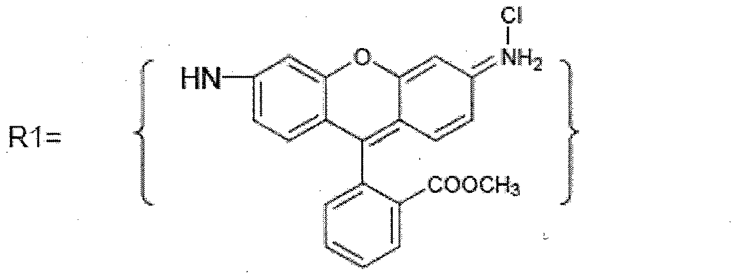
(13) hormone (estrogen) 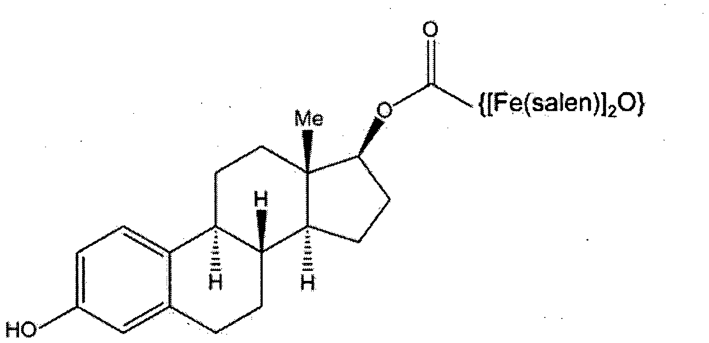
(14) hormone (estrogen) 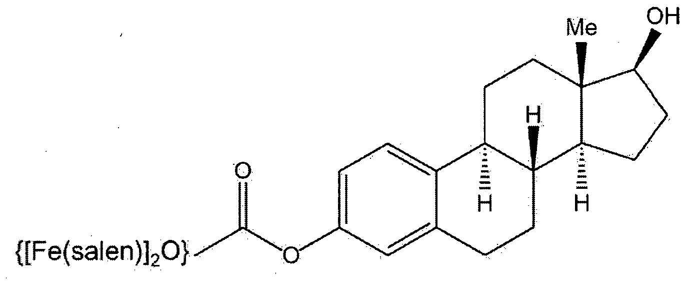
(15) Taxol (paclitaxel) 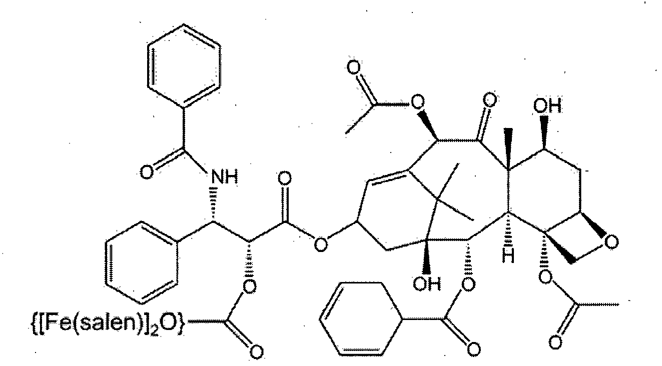
(16) amino acid (glycine) 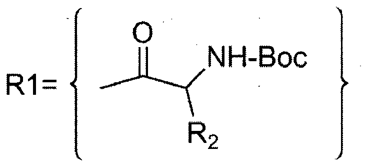
(17) amino acid (alanine) 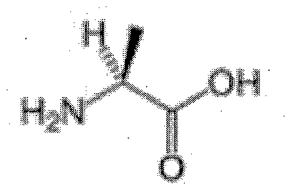
(18) amino acid (arginine) 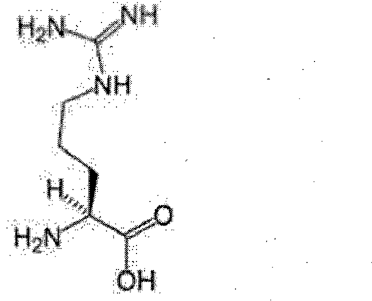
(19) amino acid (asparagine) 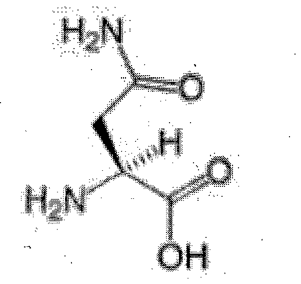
(20) amino acid (aspartic acid) 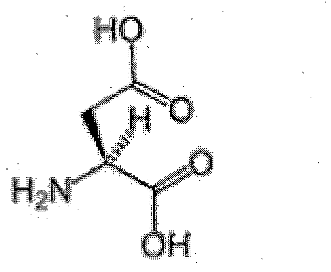
(21) amino acid (cysteine) 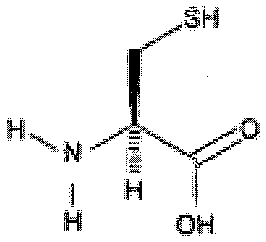
(22) amino acid (glutamic acid) 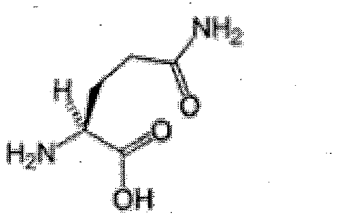
(23) amino acid (histidine) 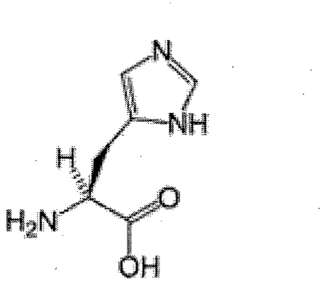
(24) amino acid (isoleucine) 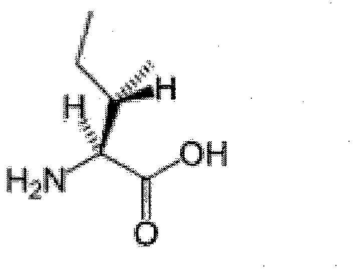
(25) amino acid (leucine) 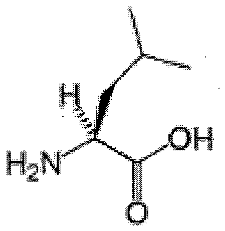
(26) amino acid (lysine) 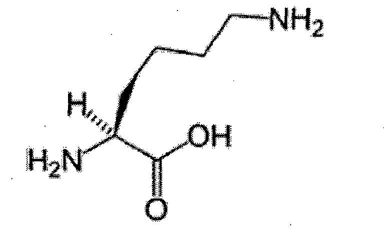
(27) amino acid (methionine) 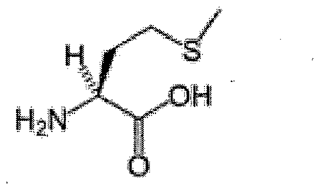
(28) amino acid (phynylalanine) 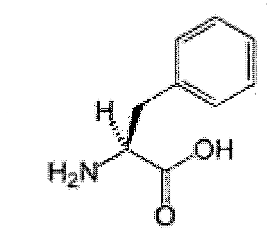
(29) amino acid (proline) 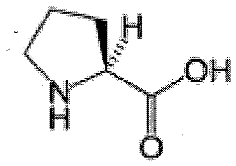
(30) amino acid (serine) 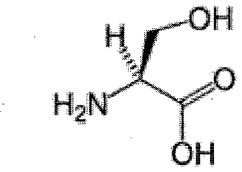
(31) amino acid (threonine) 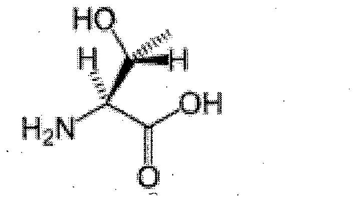
(32) amino acid (tryptophan) 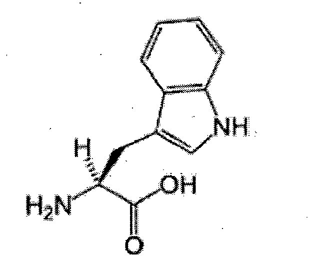
(33) amino acid (tyrosine) 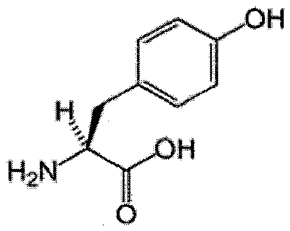
(34) amino acid (valine)

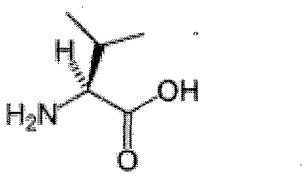

7. The self-magnetic metal-salen complex compound according to claim 4 or 5, wherein R₃ is a compound composed of a substituent represented by any of the following formulas (35) to (45) obtained as a result of desorption of hydrogen from a compound which has a methyl group and whose charge transfer is less than 0.5 electors (e):
(35) general name: lidocaine 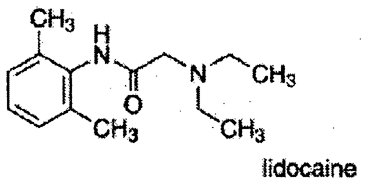
(36) general name: ethyl aminobenzoic acid 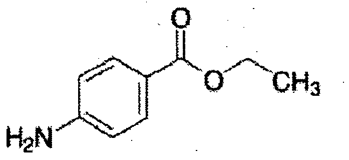
(37) general name: oxybuprocaine hydrochloride 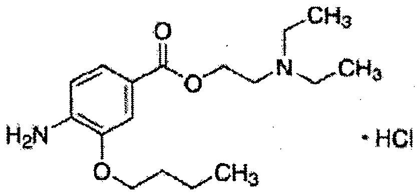
(38) general name: oxethazaine 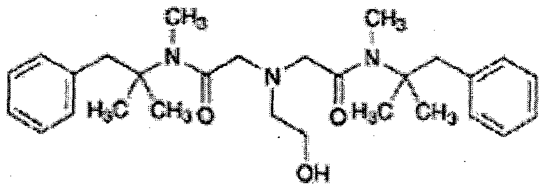
(39) general name: dibucaine 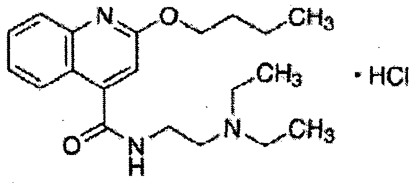
(40) general name: ethylpiperidinoacetylaminobenzoate 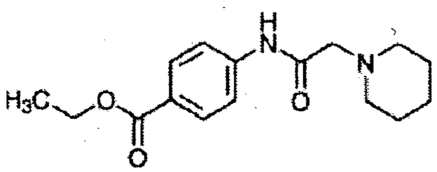
(41) general name: procaine 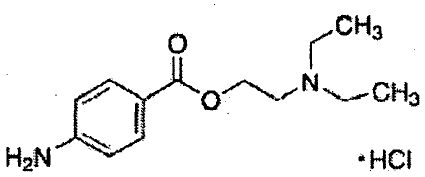
(42) general name: mepivacaine 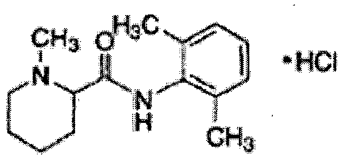
(43) general name: p-butylaminobenzoyldiethylaminoethyl hydrochloride 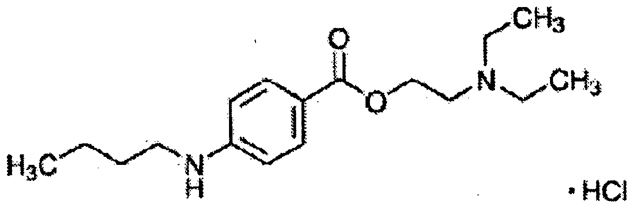
(44) general name: bupivacaine hydrochloride 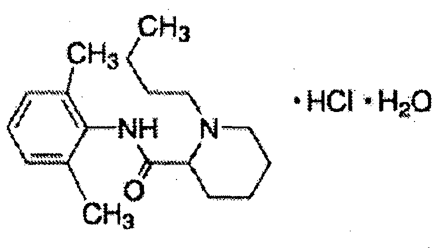
(45) general name: ropivacaine hydrochloride hydrate

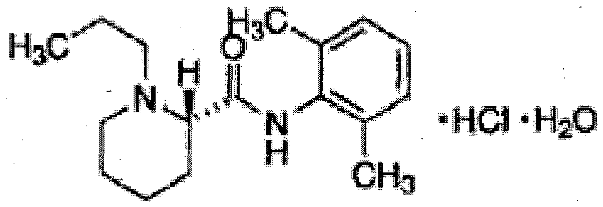

8. The self-magnetic metal-salen complex compound according to claim 4 or 5, wherein R₃ is any one of compounds represented by the following formulas (46) to (110), which combines with a main skeleton of the compound of the formula via a linking group part obtained as a result of desorption of hydrogen (however, with the compound (90), a cyano group (-CN) is the linking group):
(46) general name: ifosfamide, alkyl antineoplastic drug 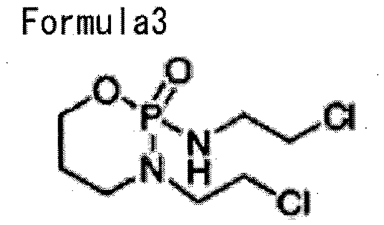
(47) general name: cyclophosphamide, alkyl antineoplastic drug 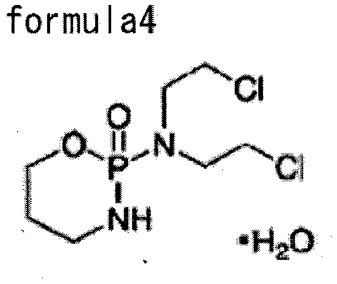
(48) general name: dacarbazine, alkyl antineoplastic drug 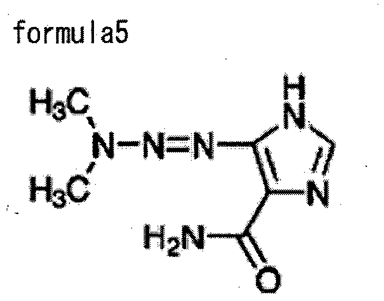
(49) general name: busulfan, alkyl antineoplastic drug 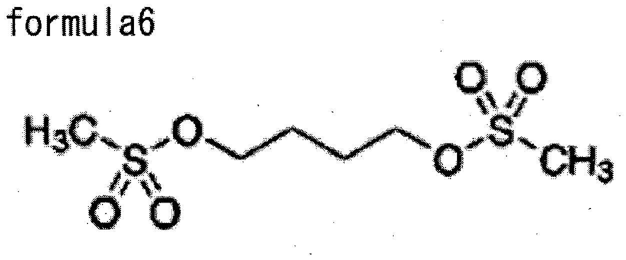
(50) general name: melphalan, alkyl antineoplastic drug 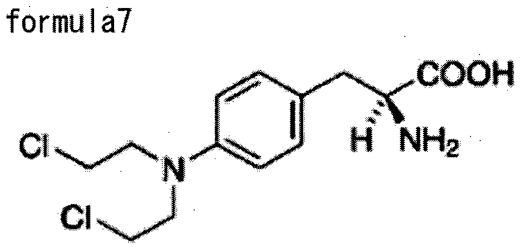
(51) general name: ranimustine, alkyl antineoplastic drug 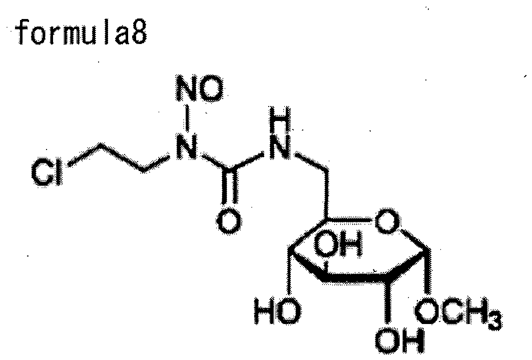
(52) general name: estramustine sodium phosphate, alkyl antineoplastic drug 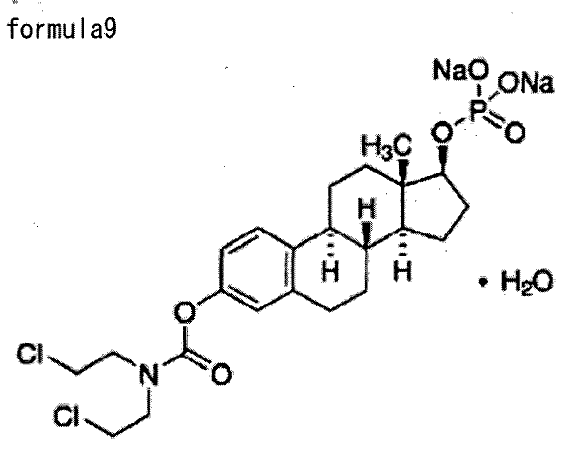
(53) general name: nimustine hydrochloride, alkyl antineoplastic drug 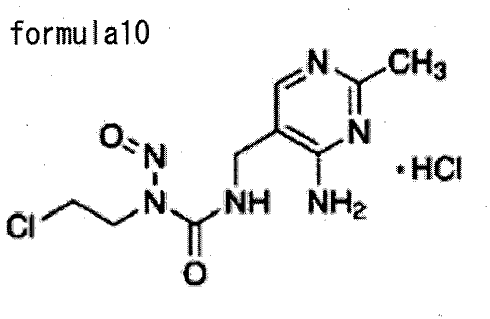
(54) general name: enocitabine, antimetabolite antineoplastic drug 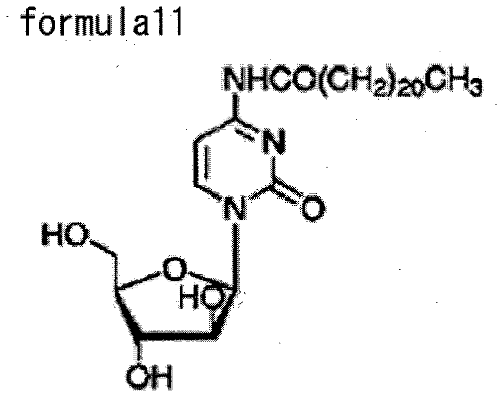
(55) general name: capecitabine, antimetabolite antineoplastic drug 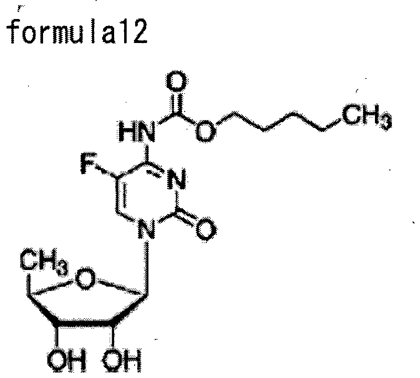
(56) general name: carmofur, antimetabolite antineoplastic drug 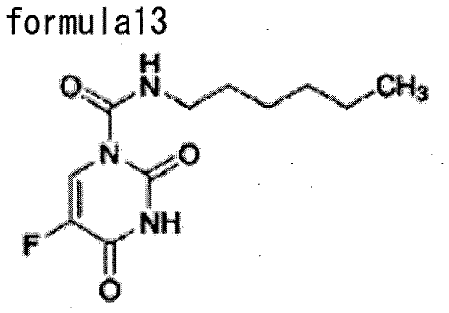
(57) general name: gimeracil, antimetabolite antineoplastic drug 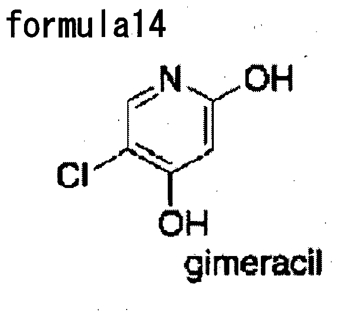
(58) general name: oteracil potassium, antimetabolite antineoplastic drug 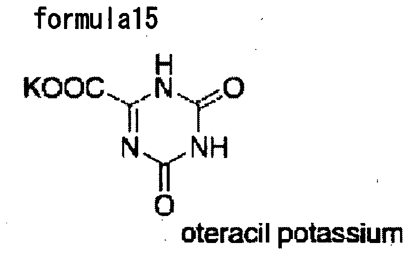
(59) general name: cytarabine, antimetabolite antineoplastic drug 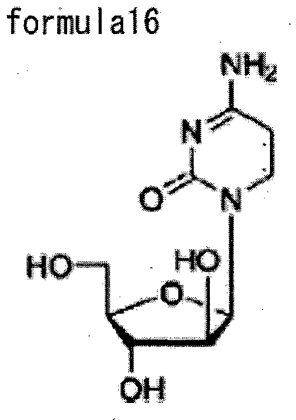
(60) general name: cytarabine ocfosfate, antimetabolite antineoplastic drug 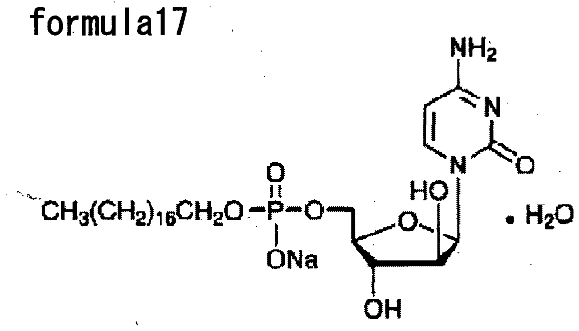
(61) general name: tegafur, antimetabolite antineoplastic drug 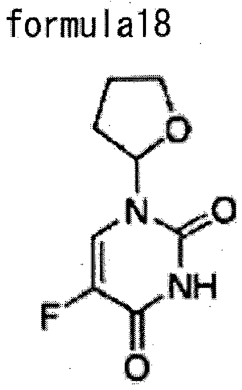
(62) general name: doxifluridine, antimetabolite antineoplastic drug 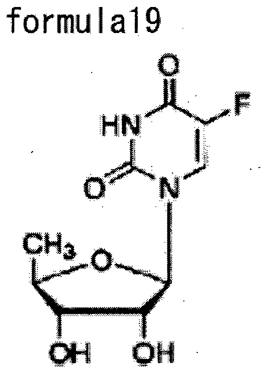
(63) general name: hydroxycarbamide, antimetabolite antineoplastic drug 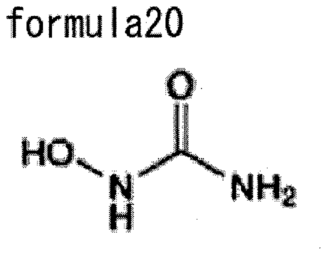
(64) general name: fluorouracil, antimetabolite antineoplastic drug 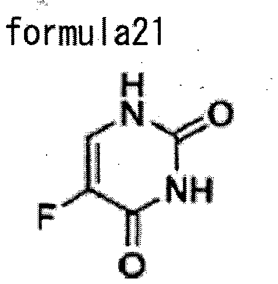
(65) general name: mercaptopurine hydrate, antimetabolite antineoplastic drug 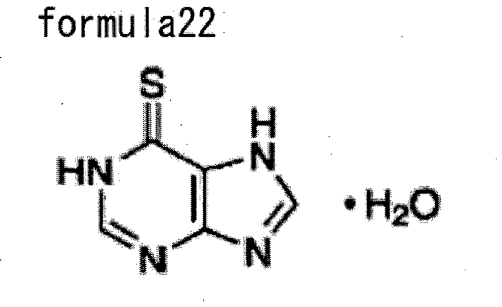
(66) general name: fludarabine phosphate, antimetabolite antineoplastic drug 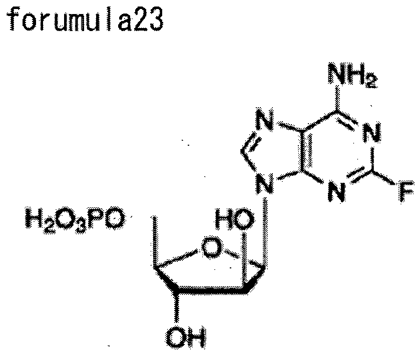
(67) general name: gemcitabine hydrochloride, antimetabolite antineoplastic drug 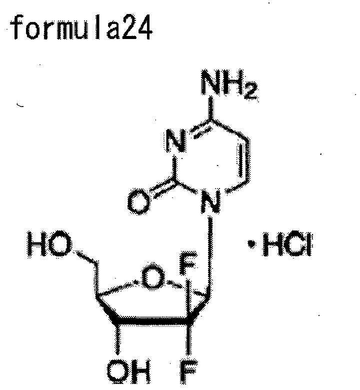
(68) general name: actinomycin-D, antitumor antibiotic 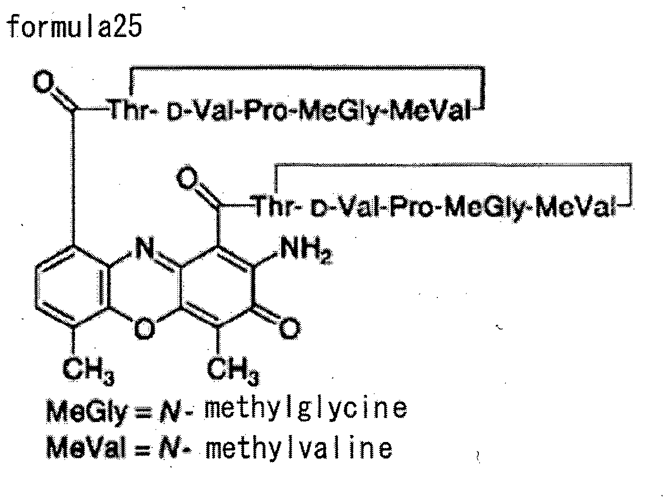
(69) general name: aclarubicin hydrochloride, antitumor antibiotic 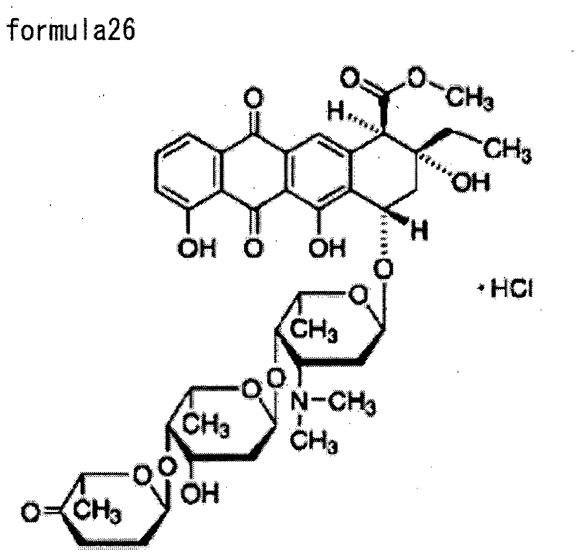
(70) general name: idarubicin hydrochloride, antitumor antibiotic 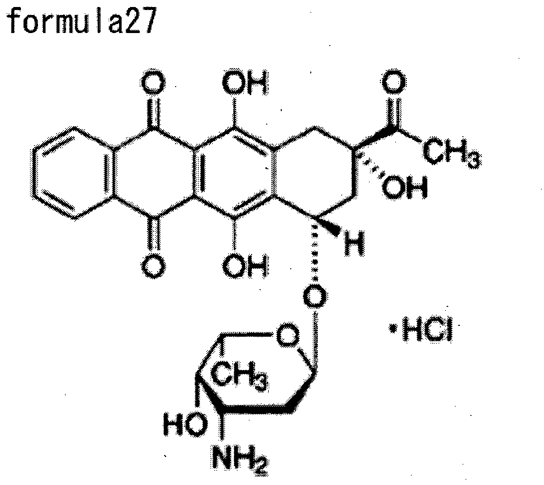
(71) general name: epirubicin hydrochloride, antitumor antibiotic 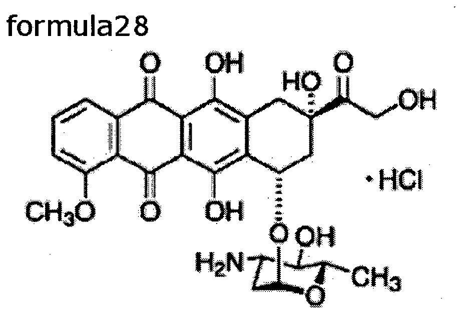
(72) general name: zinostatin stimalamer, antitumor antibiotic 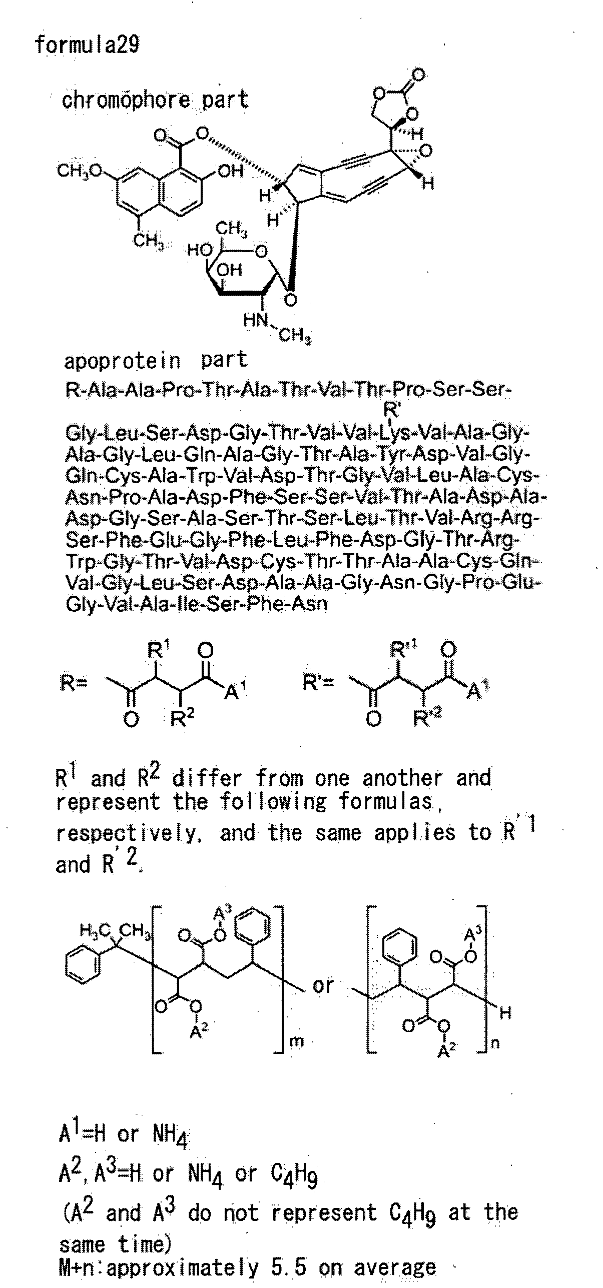
(73) general name: daunorubicin hydrochloride, antitumor antibiotic 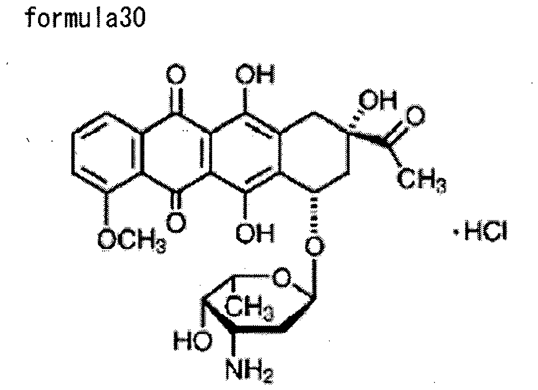
(74) general name: doxorubicin hydrochloride, antitumor antibiotic 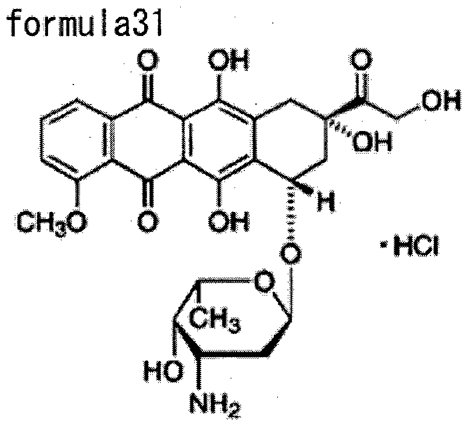
(75) general name: bleomycin hydrochloride, antitumor antibiotic 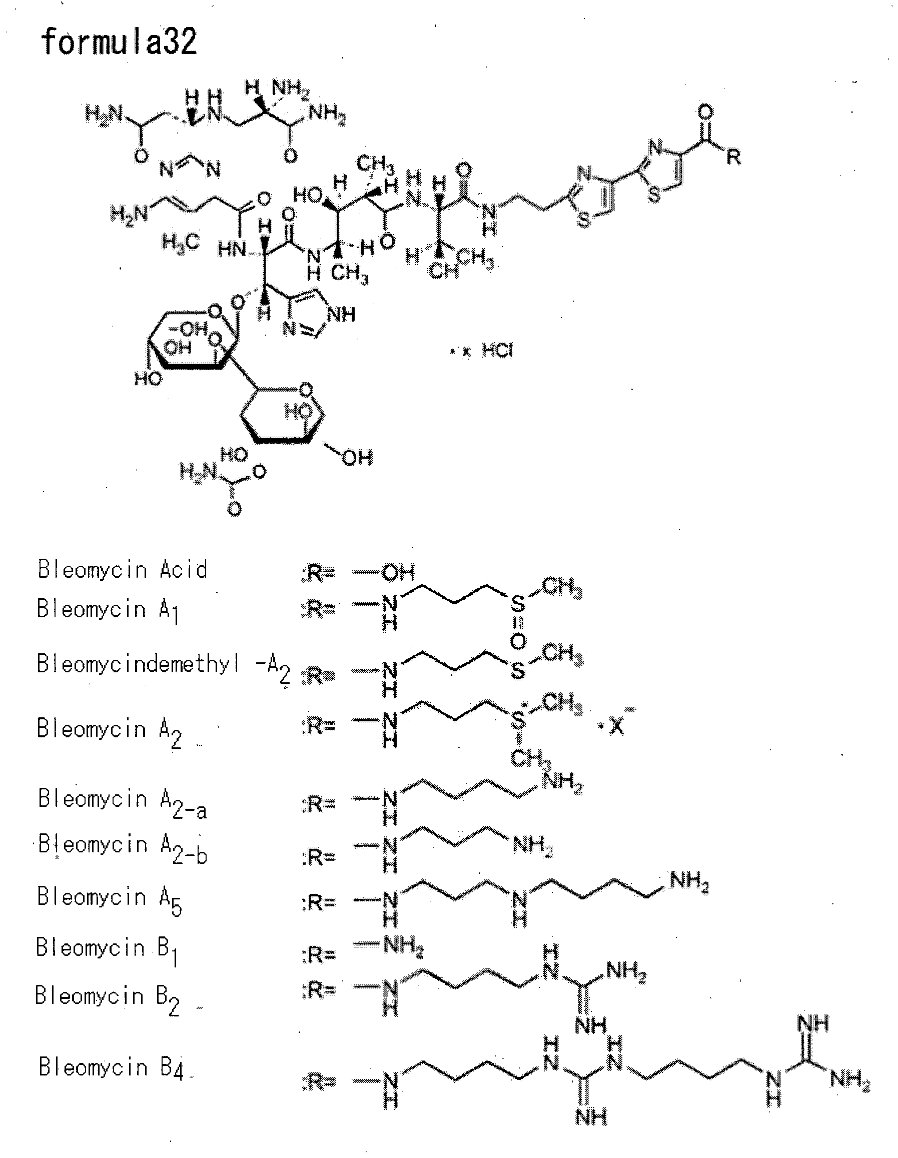
(76) general name: peplomycin sulfate, antitumor antibiotic 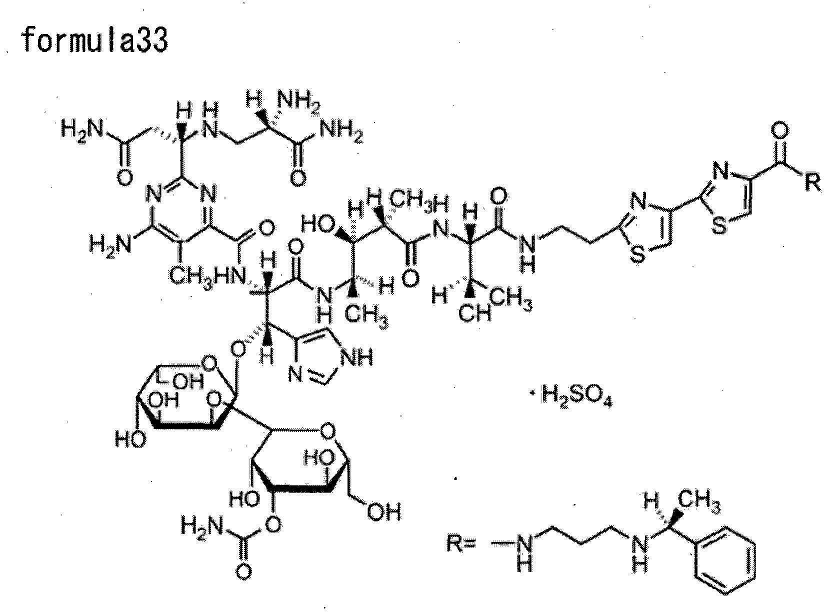
(77) general name: mitomycin C, antitumor antibiotic 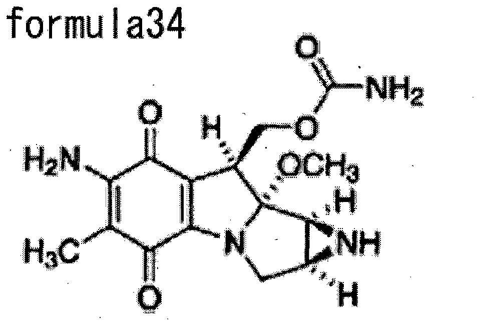
(78) general name: amrubicin hydrochloride, antitumor antibiotic 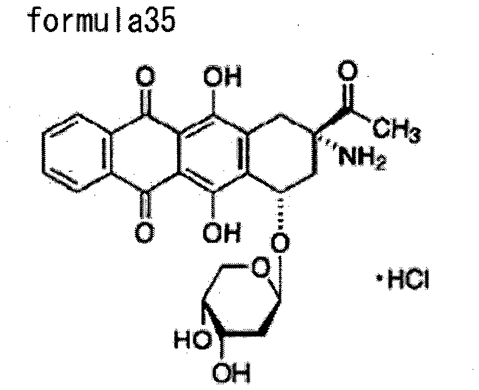
(79) general name: vibramycin hydrochloride, antitumor antibiotic 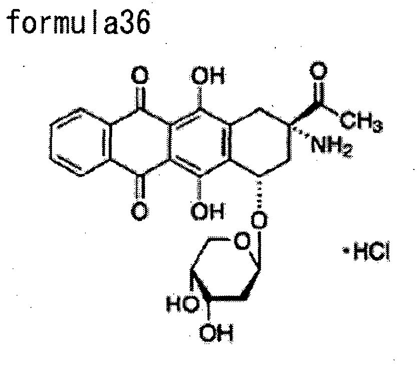
(80) general name: pirarubicin hydrochloride, antitumor antibiotic 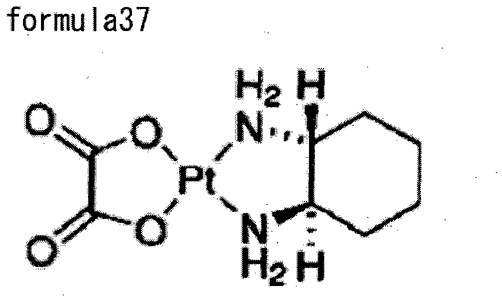
(81) general name: docetaxel hydrate, microtubule inhibitor 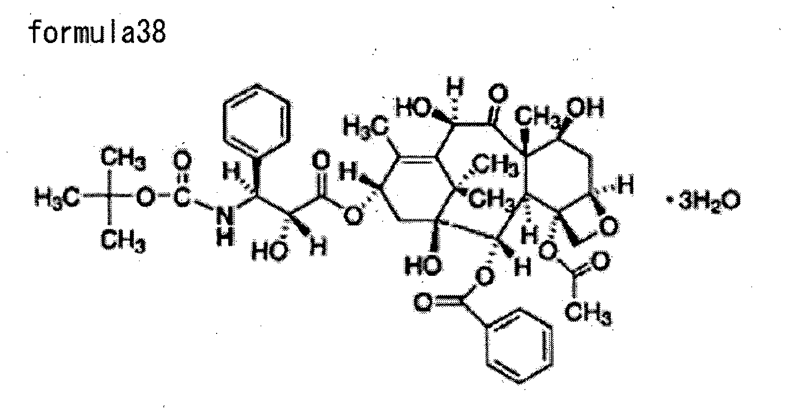
(82) general name: vincristine sulfate, microtubule inhibitor 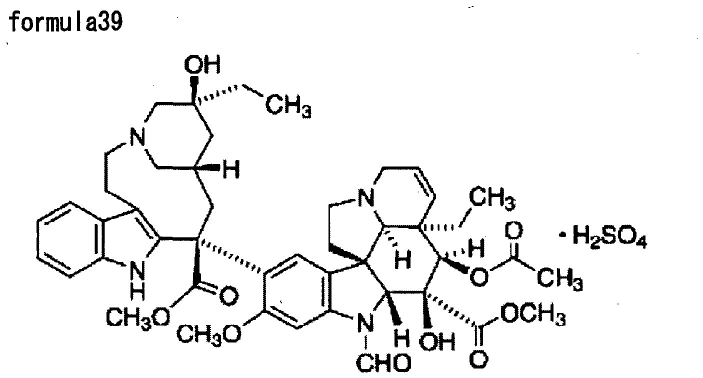
(83) general name: vinblastine sulfate, microtubule inhibitor 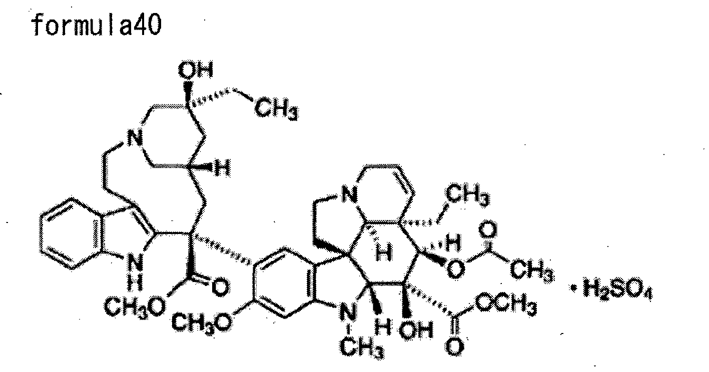
(84) general name: vinorelbine ditartrate, microtubule inhibitor 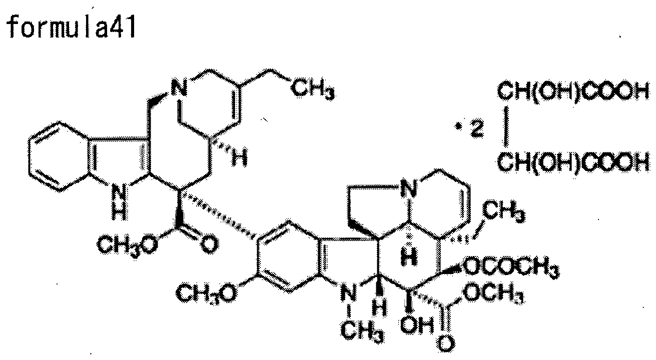
(85) general name: vindesine sulfate, microtubule inhibitor 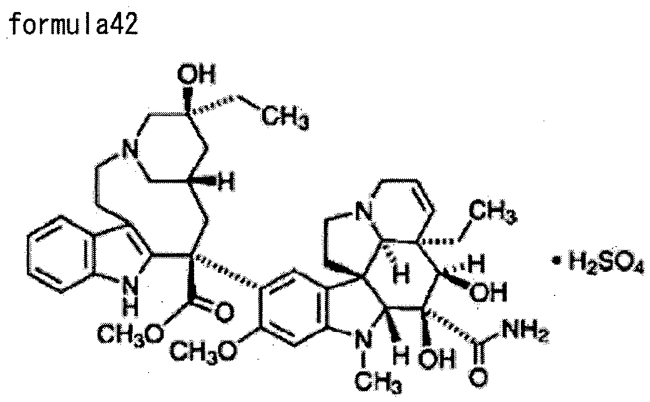
(86) general name: oxalipatin, platinum preparation 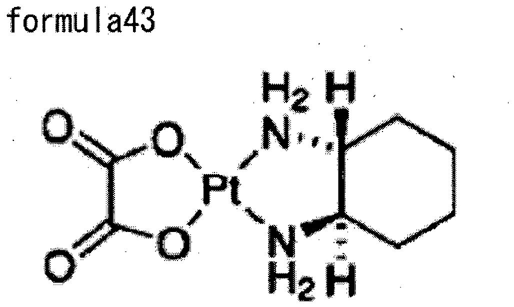
(87) general name: carboplatin, platinum preparation 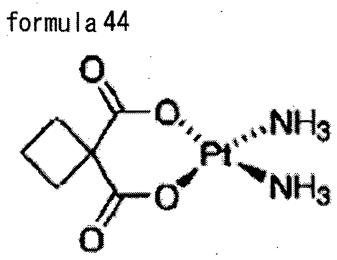
(88) general name: cisplatin, platinum preparation 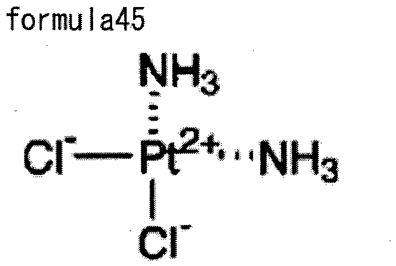
(89) general name: nedaplatin, platinum preparation 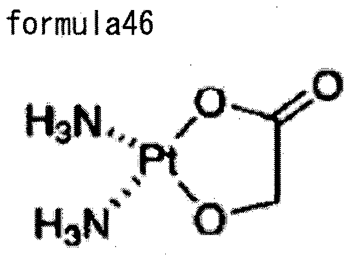
(90) general name: anastrozole, parahormone drug 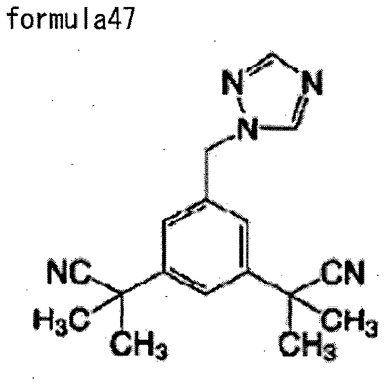
(91) general name: Afema, parahormone drug 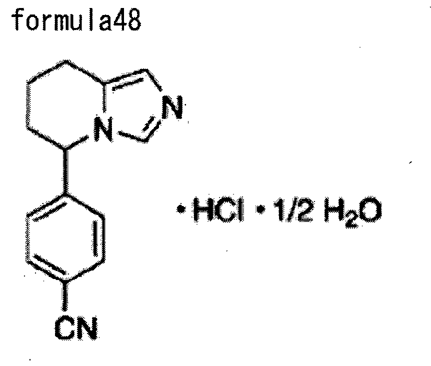
(92) general name: exemestane, parahormone drug 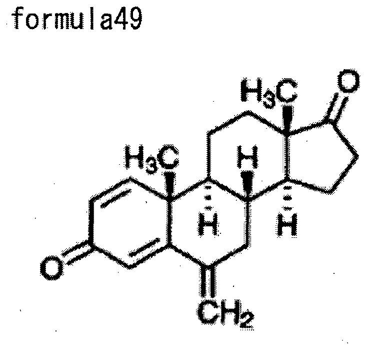
(93) general name: tamoxifen citrate, parahormone drug 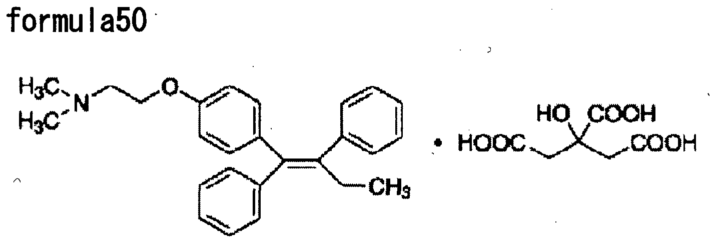
(94) general name: toremifene citrate, parahormone drug 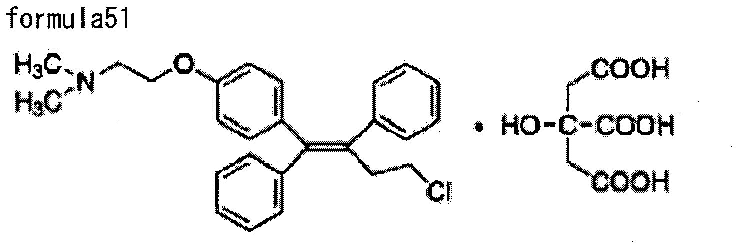
(95) general name: bicalutamide, parahormone drug 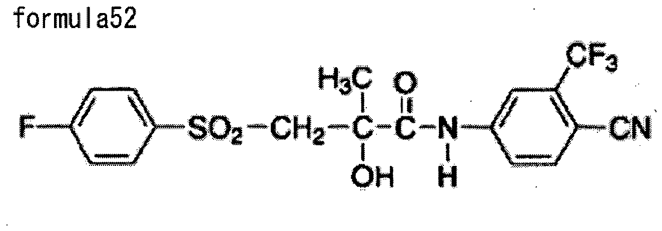
(96) general name: flutamide, parahormone drug 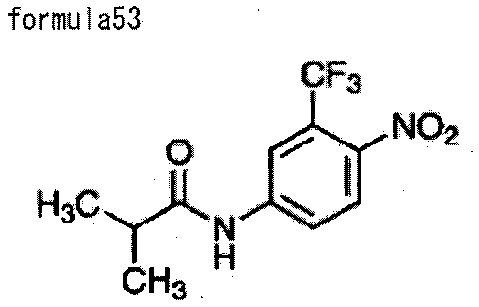
(97) general name: mepitiostane, parahormone drug 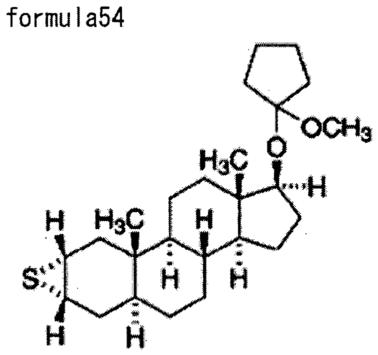
(98) general name: estramustine sodium phosphate, parahormone drug 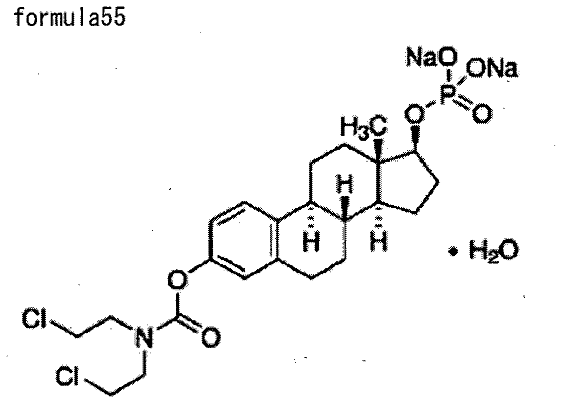
(99) general name: medroxyprogesterone acetate, parahormone drug 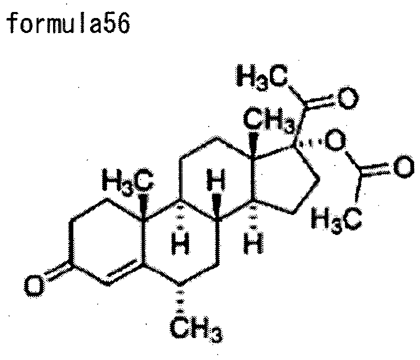
(100) general name: tamibarotene, molecular target drug 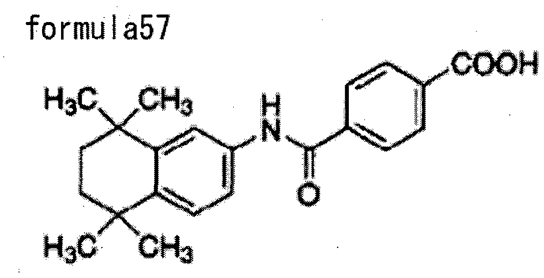
(101) general name: Gefitinib, molecular target drug 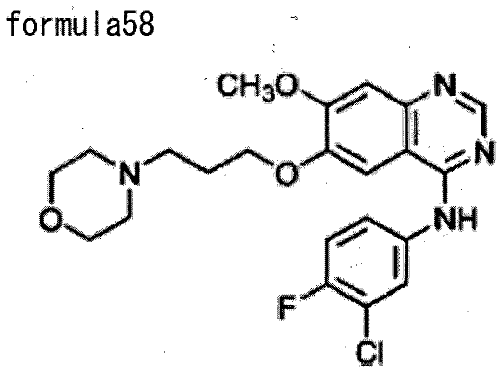
(102) general name: tretinoin, molecular target drug 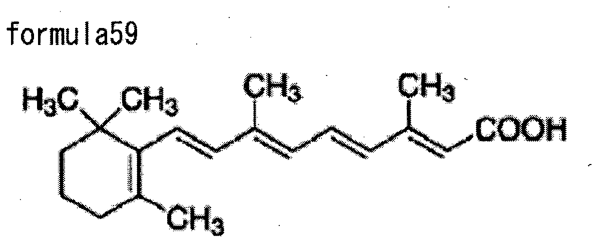
(103) general name: imatinib mesylate, molecular target drug 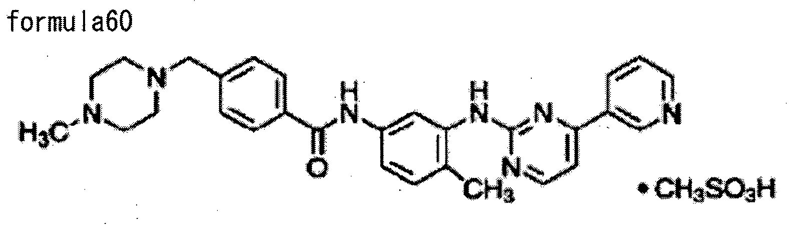
(104) general name: etoposide, topoisomerase inhibitor 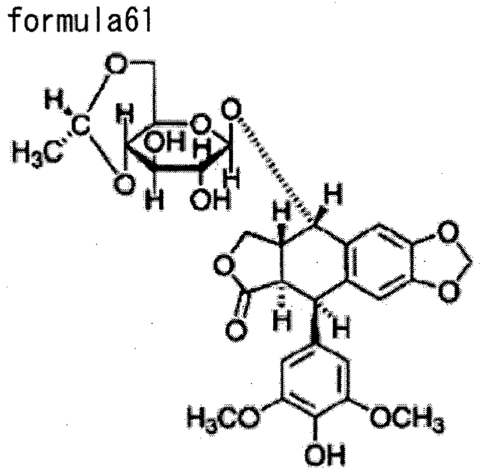
(105) general name: sobuzoxane, topoisomerase inhibitor 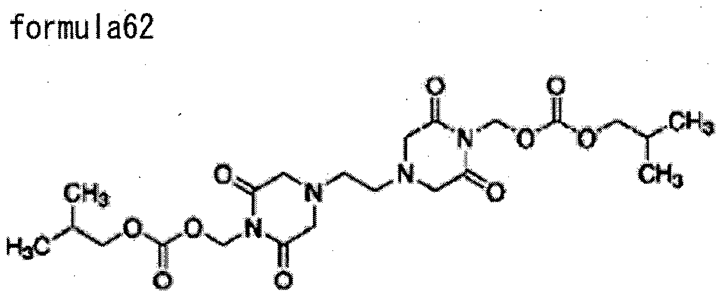
(106) general name: irinotecan hydrochloride, topoisomerase inhibitor 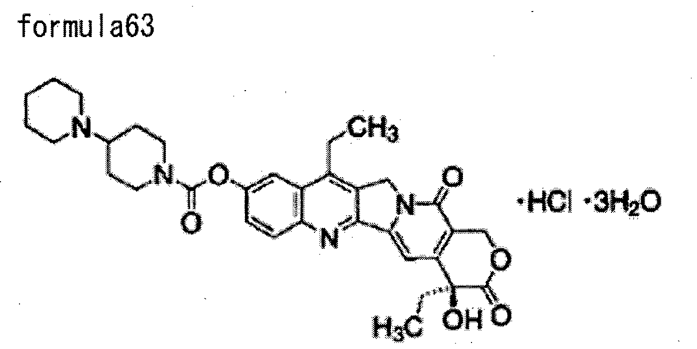
(107) general name: nogitecan hydrochloride, topoisomerase inhibitor 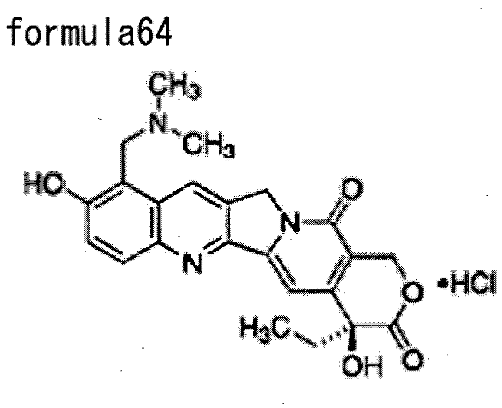
(108) general name: ubenimex, nonspecific immunopotentiator 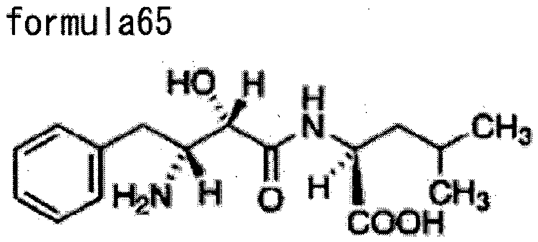
(109) general name: sizofiran, nonspecific immunopotentiator) 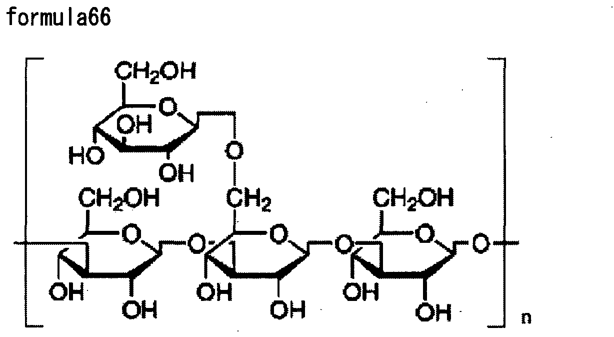
(110) general name: lenthinan, nonspecific immunopotentiator

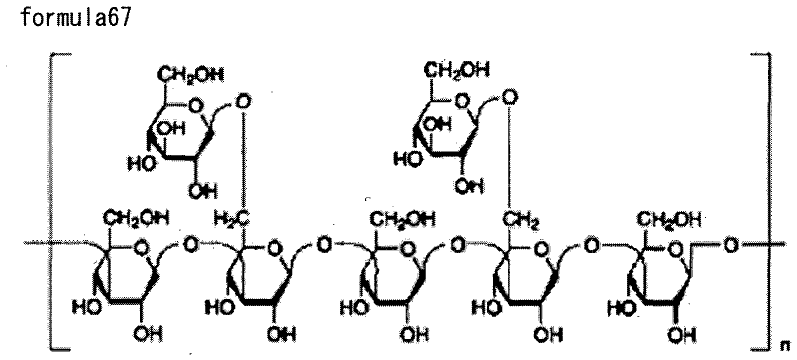

9. The self-magnetic metal-salen complex compound according to claim 4 or 5, wherein R₃ is composed of any one of compounds represented by the following formulas (111) to (116):
(111) product name: Leuplin; and general name: leuprorelin acetate, anti-tumor agent 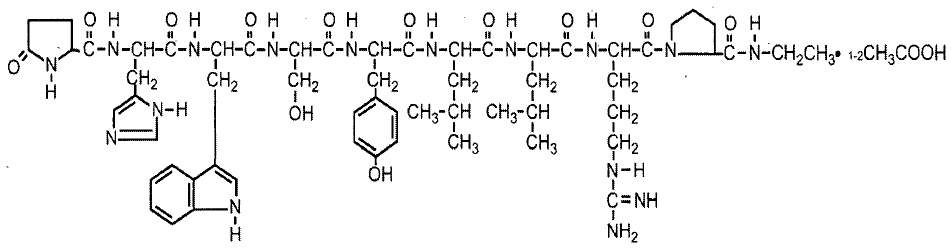
(112) product name: methotrexate; and general name: methotrexate, anti-tumor agent 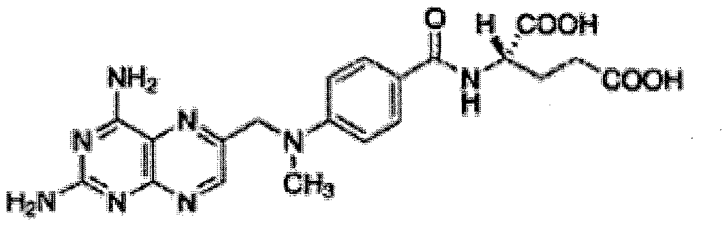
(113) product name: Novantrone; and general name: mitoxantrone hydrochloride, anti-tumor agent 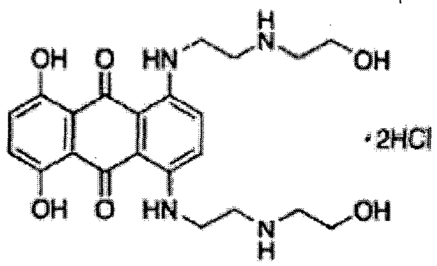
(114) product name: photofrin; and general name: porfimer sodium, anti-tumor agent 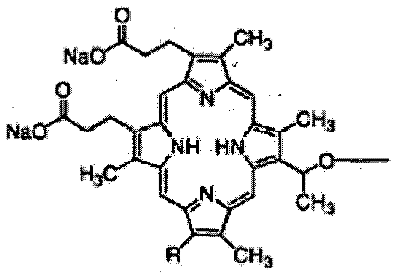
(115) product name: photofrin; and general name: porfimer sodium, anti-tumor agent 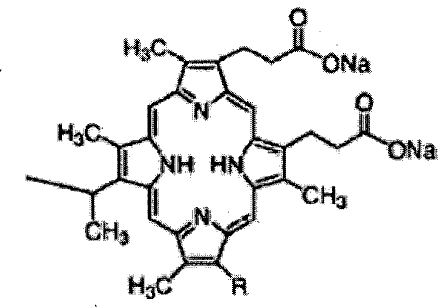
(116) product name: Mylotarg; and general name: gemtuzumab ozogamicin, anti-tumor agent.

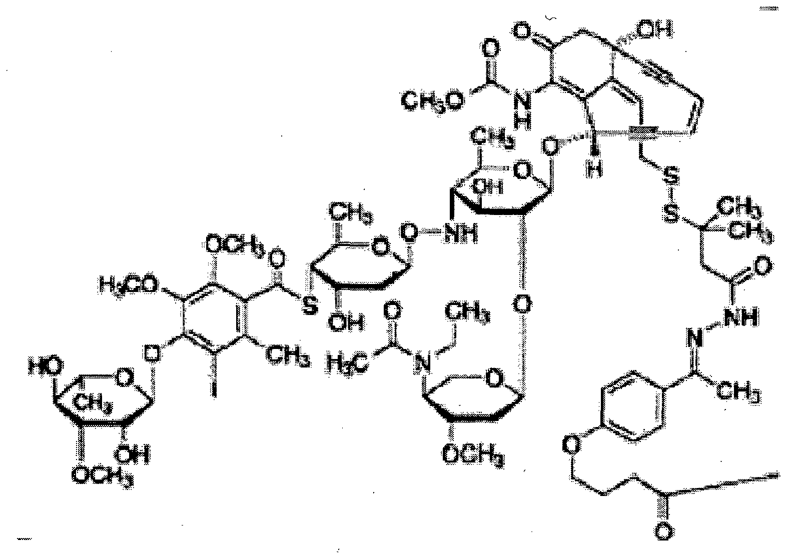

10. A self-magnetism-giving metal-salen complex molecule, wherein another compound is bonded to at least one of positions Y, a, b, c, d, e, f, g, h, i, j, k, and I and said another compound is magnetized. 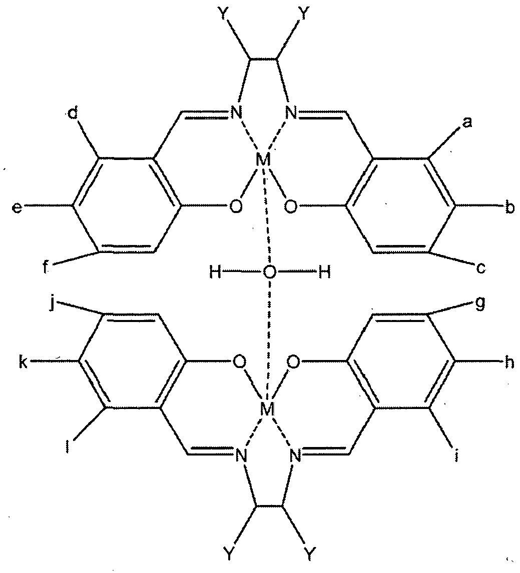 where M represents Fe, Cr, Mn, Co, Ni, Mo, Ru, Rh, Pd, W, Re, Os, Ir, Pt, Nd, Sm, Eu, or Gd.

11. A local anesthetic having the self-magnetic metal-salen complex compound stated in claim 7.

12. An antineoplastic drug having the self-magnetic metal-salen complex compound stated in claim 8.

13. An antineoplastic drug having the self-magnetic metal-salen complex compound stated in claim 9.
